# EUROPEAN PATENT APPLICATION

(11) **EP 1 378 519 A1**
(43) Date of publication of application: **07.01.2004**
(21) Application number: 02014908.4
(22) Date of filing: 05.07.2002
(51) Int. Cl.: C07K 14/705

(54) **Scianna antigens**

(71) Applicant: BIOTEST AG, D-63303 Dreieich (DE)
(72) Inventor: Flegel, Willy A., Priv.-Doz. Dr. med., 64807 Dieburg (DE); Wagner, Franz F., Priv.-Doz. Dr. med., 89075 Ulm (DE)
(74) Representative: Keller, Günter, Dr.

(57) **Abstract**

The present invention relates to novel polynucleotides encoding a Scianna (Sc) and/or Radin (Rd) antigen which are characterized by one or a combination of mutations of the ERMAP gene. The present invention further relates to vectors comprising the polynucleotides of the invention, to hosts transformed or transfected with said vectors, to polypeptides encoded by said polynucleotides and to methods of producing such polypeptides. Finally, the invention relates to a kit useful for testing for the presence of Scianna antigens.

## Description

The present invention relates to novel polynucleotides encoding a Scianna and/or Radin antigen which are characterized by one or a combination of mutations of the ERMAP gene. The present invention further relates to vectors comprising the polynucleotides of the invention, to hosts transformed or transfected with said vectors, to polypeptides encoded by said polynucleotides and to methods of producing such polypeptides. The fact that the mutations in the ERMAP gene referred to above can be correlated to Scianna antigens has a significant impact on the testing of blood samples and on blood group determination. For example, oligonucleotides and antibodies can now be designed that generally allow the detection of the Scianna blood group of a sample. Such oligonucleotides, antibodies as well as several diagnostic methods fall within the scope of the present invention. Scianna antigens encoded by the polynucleotides of the invention may be used for the characterization, standardization and quality control of monoclonal and polyclonal antisera. Finally, the invention relates to a kit useful for testing for the presence of Scianna antigens.

The molecular characterization of polymorphism underlying blood group antigens has made enormous advances during the last 15 years (Cartron and Colin, 2001). Currently, 26 blood group systems are recognized (Daniels et al., 2001). The antigens of 22 blood group systems are determined by epitopes on proteins. They may function as transport or adhesion proteins, ectoenzymes, or cytokine receptors present on the red blood cells (RBCs) surface (Cartron and Colin, 2001). Most recently, the *DOK1* gene determining the Dombrock blood group system (ISBT 014) had been characterized (Gubin et al., 2000), leaving Scianna (ISBT 013) the last protein-based blood group system whose molecular basis remained obscure.

The Scianna blood group system comprises two antithetical antigens, the high-frequency antigen Sc1 (ISBT 013.001, formerly Sm ) and the low frequency antigen Sc2 (ISBT 013.002, formerly Bu). Lewis et al. recognized the relationship of the antigens and coined the name Scianna (Lewis et al., 1974; Issitt and Anstee, 1998). The antigen Sc3 (ISBT 013.003) is expressed on all RBCs except those of the very rare Sc:-1,-2 phenotype, three clusters of which were observed in circumscribed populations (Issitt and Anstee, 1998).

The Scianna antigens were shown to reside on a "Scianna glycoprotein" of about 60-68 kD (Spring et al., 1990). Scianna antigens were inherited linked to the RH antigens, but shown to be distinct (Lewis et al., 1976). The latest data indicated a genomic position of 1p34 to 1p36 (Reid and Lomas-Francis, 1997). A second low-frequency antigen, Rd (700.015) shared this position (Lewis et al., 1980; Hilden et al., 1985) and was found on a protein of similar size (Spring, 1993). Both anti-Sc2 (DeMarco et al., 1995) and anti-Rd (Rausen et al., 1967) are clinically relevant, having caused hemolytic disease of the newborn. The molecular basis and function of the Scianna blood group system and its glycoprotein remained unknown.

It has been found by the inventors of the present invention that the Scianna antigens are expressed by the red cell adhesion protein ERMAP. The polymorphism underlying the Sc2 and Rd antigens were determined. PCR-SSP (= polymerase chain reaction with sequence specific priming) methods that allow the genotypic prediction of these antigens were developed.

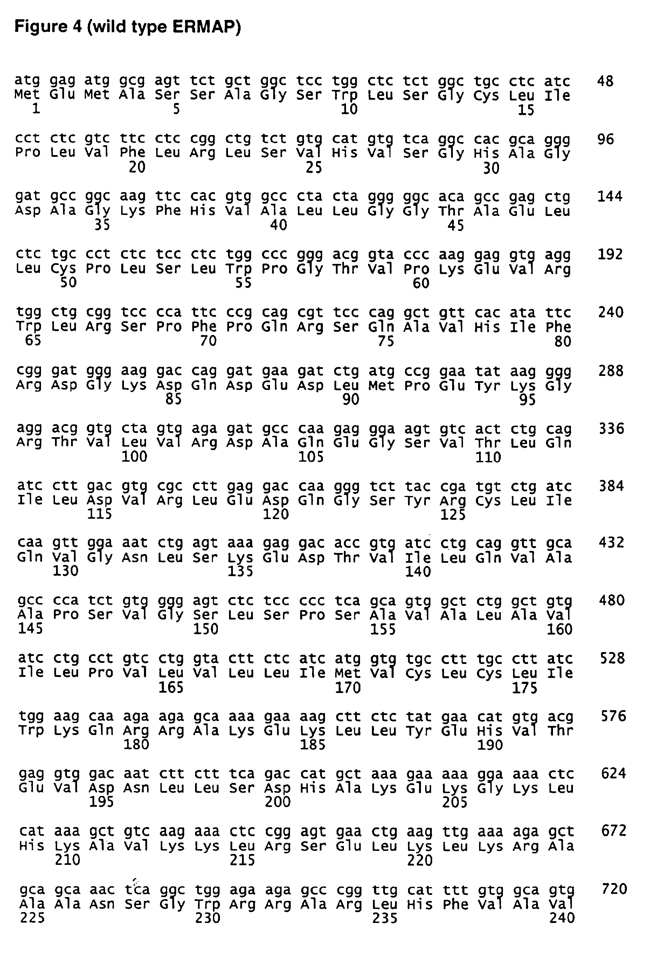

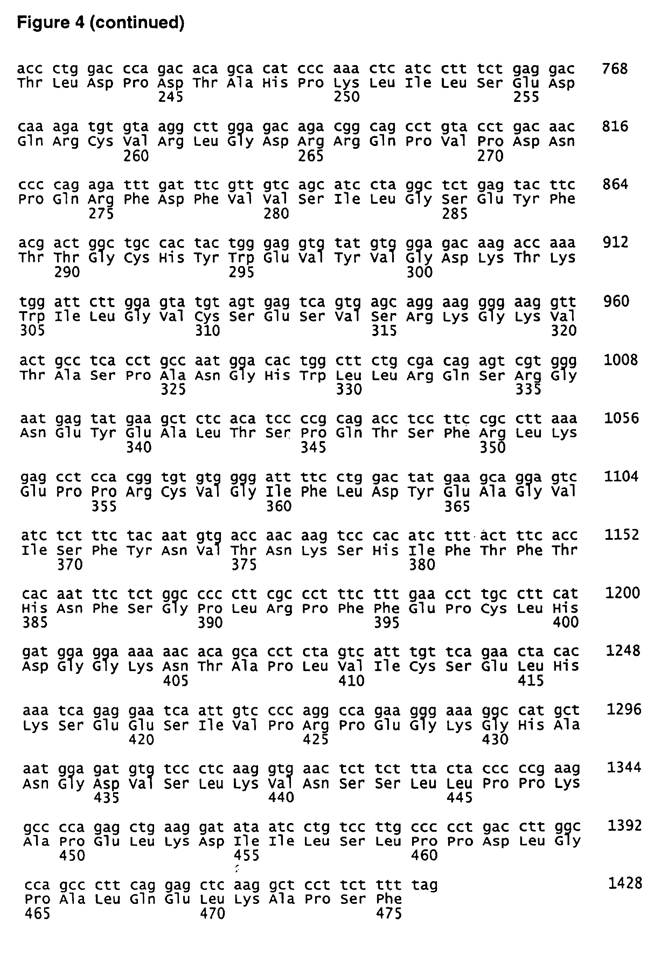

The red cell adhesion protein ERMAP had been identified by Xu et al. (2001). The published nucleotide sequence (SEQ ID NO:1) encodes a membrane protein of 475 amino acids (SEQ ID NO:2; see Figure 4). Since the first 29 amino acids are believed to constitute the signal peptide, the mature protein presumably has a length of 446 amino acids. Amino acids 30 to 156 are predicted to form the extracellular portion of ERMAP.

In a first aspect the present invention relates to a polynucleotide encoding human ERMAP or a variant of human ERMAP or a fragment of human ERMAP or of said variant, wherein said polynucleotide carries at least one mutation as compared to the nucleotide sequence encoding human wild type ERMAP. The nucleotide sequence encoding human wild type ERMAP is shown in SEQ ID NO:1 (See also figure 4).

The mutant polynucleotides referred to above and further throughout this specification can be conveniently employed for the characterization of monoclonal and polyclonal antibodies used in connection with Scianna antigen determination. For example, by expressing desired polynucleotides encoding such mutants in a suitable system, reactivity profiles of said antibodies or antisera can be established. The mutants can also be employed for the characterization of monoclonal and polyclonal antibodies that are used as secondary antibodies, for example, anti-globulin and anti-human antisera. As used herein, the term "Scianna antigen" comprises Scianna antigens and the Radin antigen.

As used herein, the term "mutation" denotes any change in the nucleotide or amino acid sequence of a given wild type sequence. The term "mutation" encompasses silent mutations which do not cause amino acid substitutions in the encoded protein, missense mutations which cause amino acid substitutions in the encoded protein, nonsense mutations, deletions, insertions, inversions etc. The number of mutations in the polynucleotide as compared to the wild type sequence as shown in SEQ ID NO:1 is at least one. Preferably, the number of mutations is not higher than 10, more preferably not higher than 5, most preferably not higher than 3. The most preferred polynucleotides of the invention carry 1 to 3 mutations as compared to the wild type sequence as shown in SEQ ID NO:1. A deletion or insertion of two or more consecutive nucleotides is to be understood as one mutation.

The fragments of human ERMAP or of the ERMAP variant preferably have a length of at least 7 amino acids, more preferably of at least 10 amino acids, even more preferably of at least 15 amino acids, most preferably of at least 25 amino acids. Correspondingly, the polynucleotide of the invention preferably comprises a coding sequence having a length of at least 21 nucleotides, more preferably at least 30 nucleotides, even more preferably at least 45 nucleotides, most preferably at least 75 nucleotides. In one embodiment, the polypeptide encoded by the polynucleotide of the invention has a length of 446 or 475 amino acids, corresponding to the mature ERMAP protein and the preprotein, respectively. In a preferred embodiment, the fragment of human ERMAP or of the ERMAP variant is an antigenic fragment.

As used herein, the term "antigenic fragment" denotes a fragment of a polypeptide which is capable of inducing the formation of antibodies when introduced into an animal, e.g. into a rabbit. Methods of testing, whether a peptide or a fragment of a polypeptide induces the formation of antibodies are known to those skilled in the art.

Preferably, the mutation concerns a nucleotide which encodes an amino acid corresponding to an amino acid comprised in the extracellular portion of the human ERMAP protein, i.e. amino acid positions 30 to 156 of the amino acid sequence of human wild type ERMAP (SEQ ID NO:2). It is also preferred that the mutation is a missense mutation or a deletion. In one embodiment of the invention, the mutation is a missense mutation causing an amino acid substitution in the extracellular portion of the encoded protein as compared to the wild type ERMAP protein. Unless otherwise indicated, nucleotide or amino acid positions in the present application refer to the numbering of the wild type sequence of ERMAP as shown in Figure 4.

Preferably, the mutation causes an amino acid substitution in position 26, 57 or 60 or a combination of/or involving said substitutions. This preferred embodiment, besides the single mutations indicated, may comprise a combination of these substitutions. Additionally, it contemplates the possibility that one or more of said substitutions are involved, and additional mutations such as mutations leading to substitutions are present. In accordance with the present invention, it is understood that such additional mutations may be tested for when assessing the Scianna antigens in a sample. Accordingly, such embodiments reflecting the detection of additional mutations occurring in combination with the mutations identified in this application are also encompassed by the invention.

In a particularly preferred embodiment of the polynucleotide of the invention, said amino acid subsitution in amino acid position 26 is from His to Tyr, in position 57 from Gly to Arg, and/or in position 60 from Pro to Ala.

In a further preferred embodiment of the polynucleotide of the invention, said mutation occurs in nucleotide position 54, 76, 169, 178, 307 or 308 or in a combination of said positions. Particularly preferred is that said mutation is a silent mutation in nucleotide position 54 from C to T, or a missense mutation in nucleotide position 76 from C to T, in nucleotide position 169 from G to A and/or in nucleotide position 178 from C to G, and/or a deletion of nucleotide positions 307 and/or 308 of the human wild type ERMAP gene.

In the case that combinations of mutations are present, it is preferred that said combination of mutations comprises nucleotide positions 54 and 76 and is preferably C to T at position 54 and C to T at position 76; or it comprises nucleotide positions 54, 76, 307 and 308 and is preferably C to T at position 54, C to T at position 76 and a deletion of nucleotides 307 and 308 of SEQ ID NO:1.

Particularly preferred are polynucleotides comprising a nucleotide sequence selected from the group consisting of the sequences SEQ ID NO:3, SEQ ID NO:5, SEQ ID NO:6, SEQ ID NO:8 and SEQ ID NO:10. These sequences represent allelic variants of the gene encoding human ERMAP.

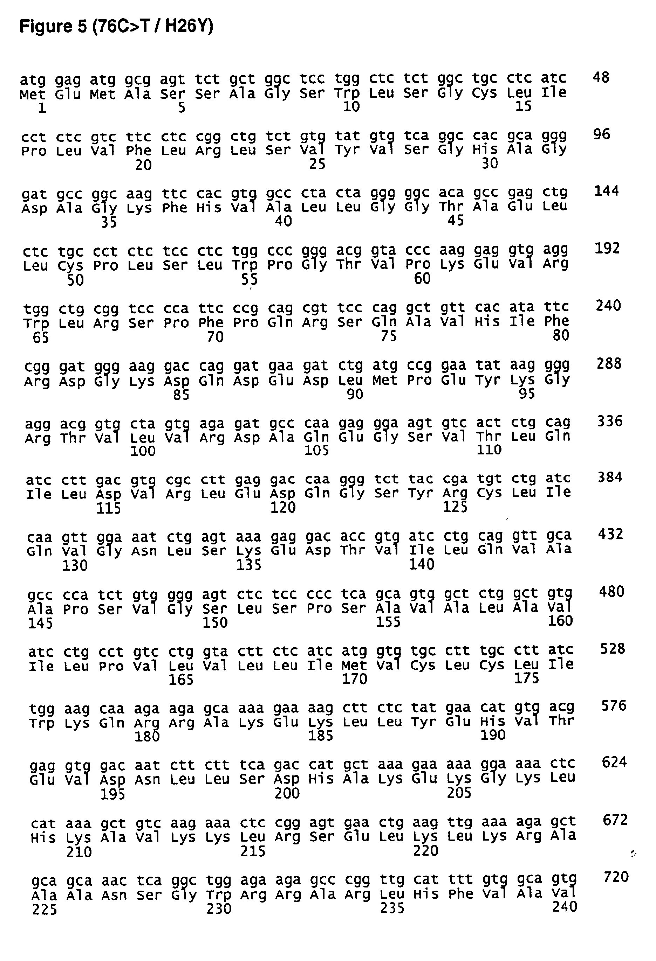

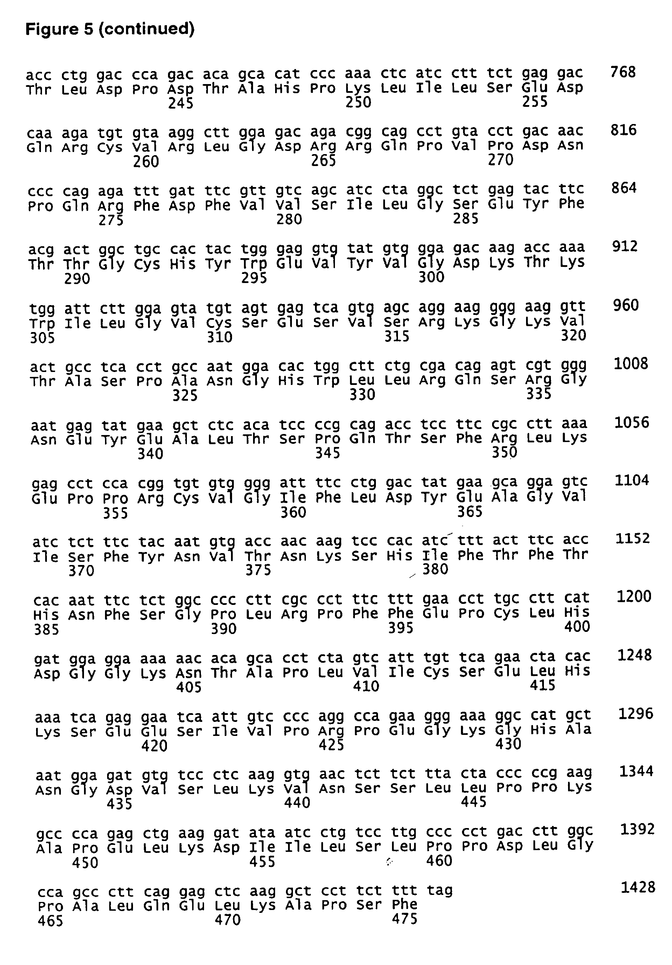

The sequence as shown in SEQ ID NO:3 carries a missense mutation at nucleotide position 76 from C to T as compared to the wild type sequence causing an amino acid substitution at position 26 from His to Tyr (See figure 5). The amino acid sequence encoded by SEQ ID NO:3 is shown in SEQ ID NO:4.

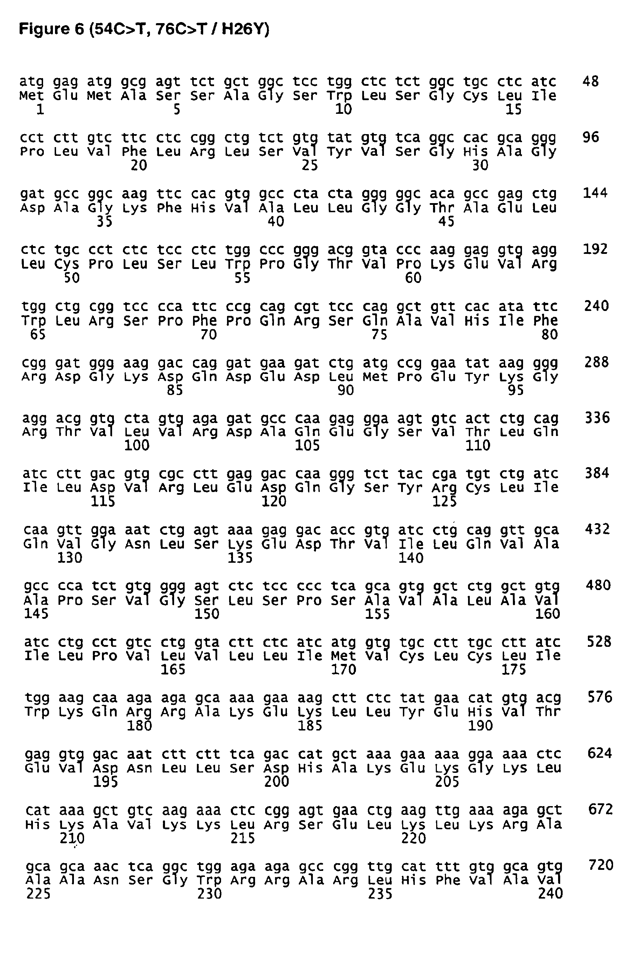

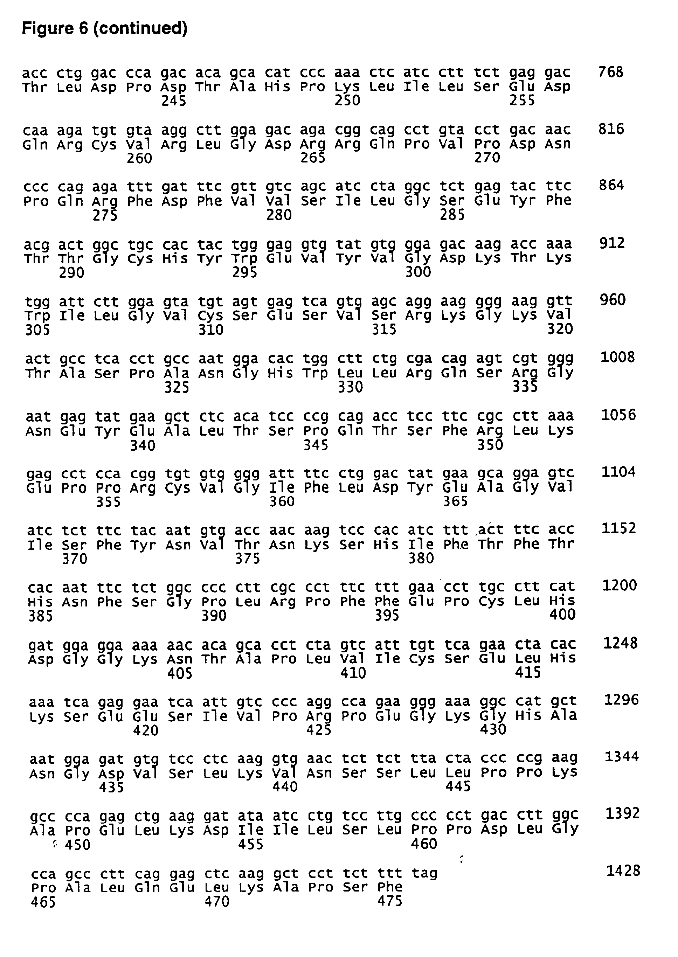

The nucleotide sequence as shown in SEQ ID NO:5 carries in addition to the 76C>T mutation a silent mutation at position 54 from C to T. The amino acid sequence encoded by SEQ ID NO:5 is therefore also shown in SEQ ID NO: 4 (See figure 6).

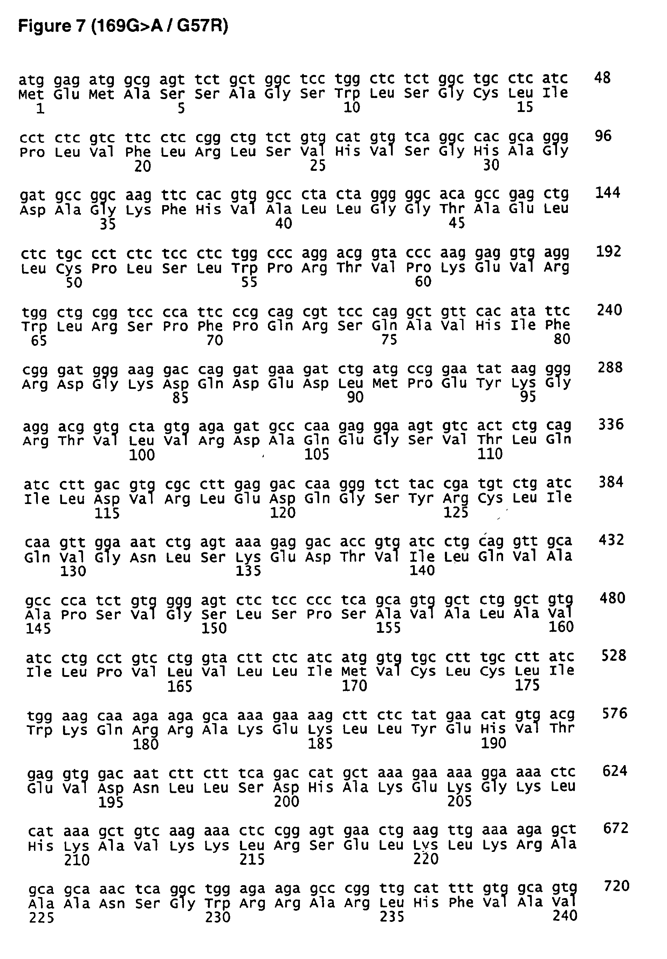

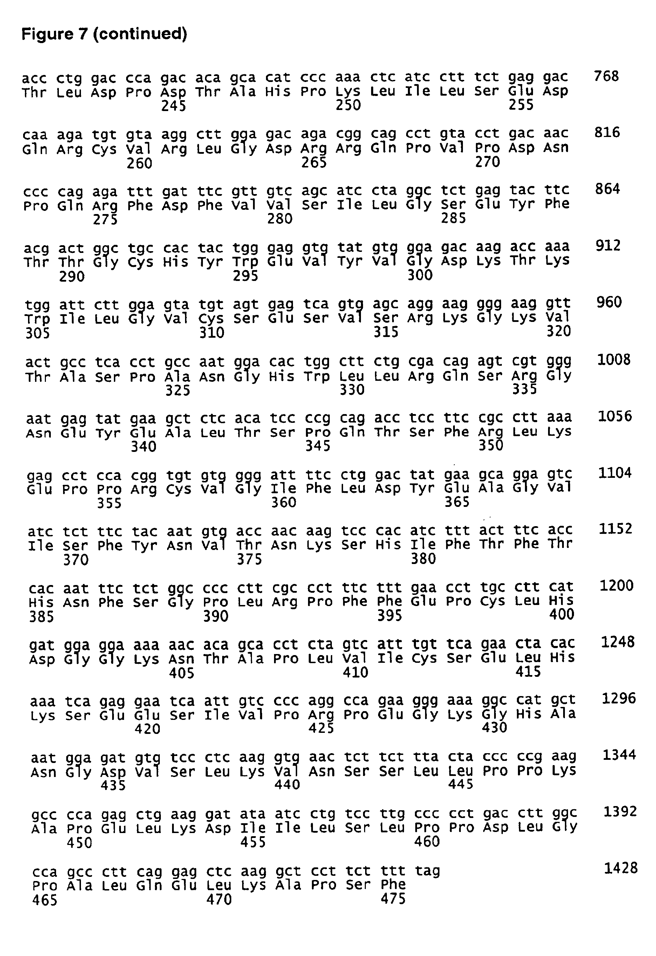

The sequence as shown in SEQ ID NO:6 carries a missense mutation at nucleotide position 169 from G to A as compared to the wild type sequence causing an amino acid substitution at position 57 from Gly to Arg (See figure 7). The amino acid sequence encoded by SEQ ID NO:6 is shown in SEQ ID NO:7.

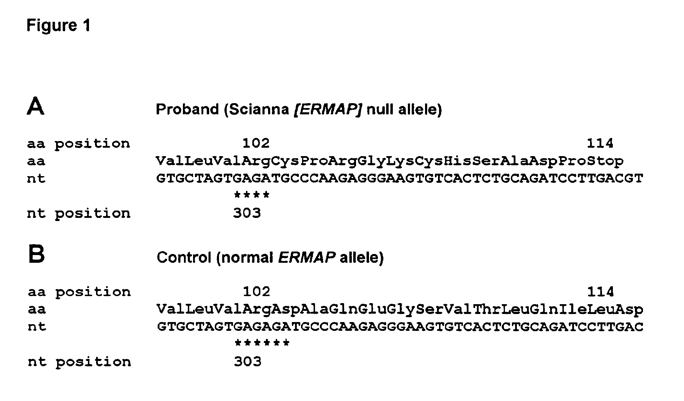

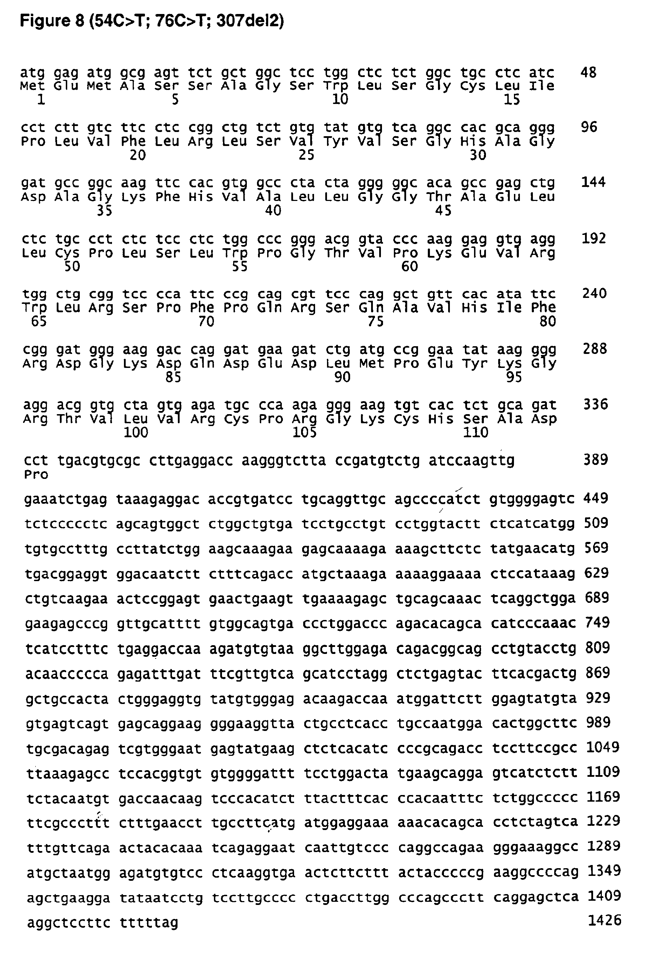

The sequence as shown in SEQ ID NO:8 has a deletion of nucleotide positions 307 and 308 of the wild type sequence causing a shift in the reading frame and a truncated protein (See figures 1 and 8). The amino acid sequence encoded by SEQ ID NO:8 is shown in SEQ ID NO:9.

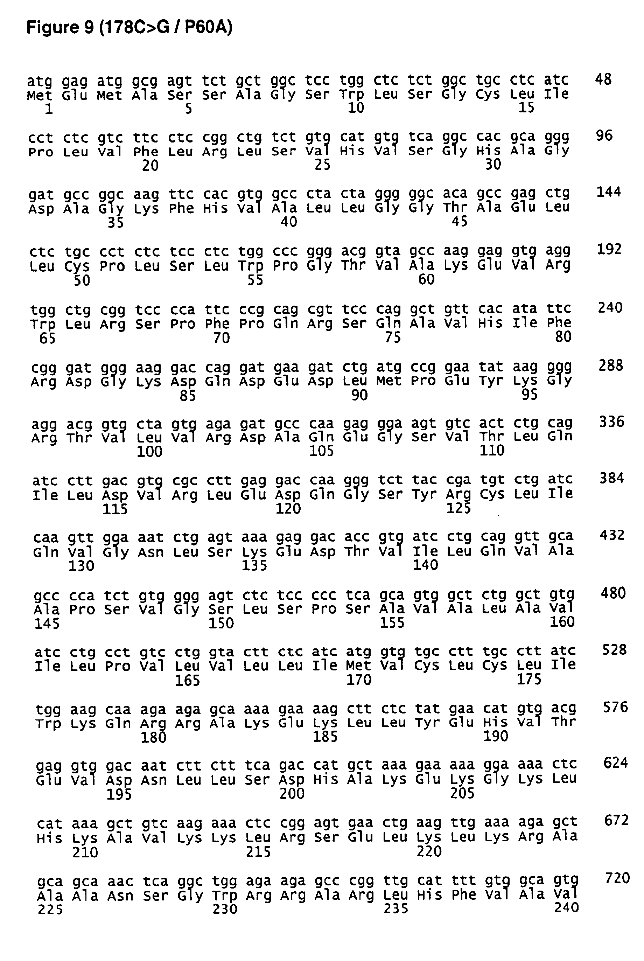

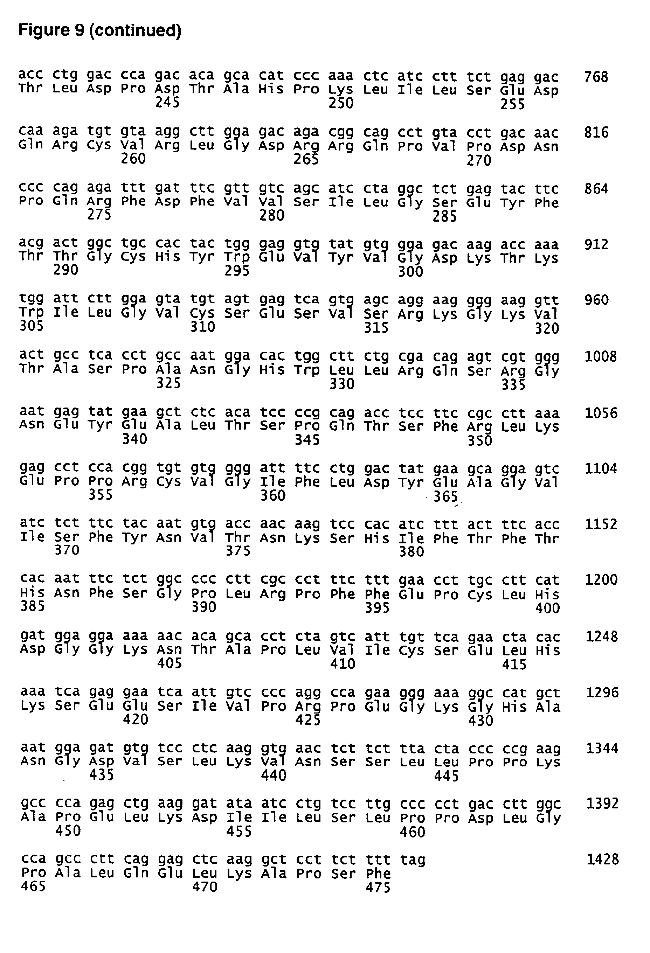

The sequence as shown in SEQ ID NO:10 carries a missense mutation at nucleotide position 178 from C to G as compared to the wild type sequence causing an amino acid substitution at position 60 from Pro to Ala (See figure 9). The amino acid sequence encoded by SEQ ID NO: 10 is shown in SEQ ID NO: 11.

The invention does not cover the DNA molecule the sequence of which is obtainable from Genbank under Accession AK056138 (Homo sapiens cDNA FLJ31576 fis, clone NT2R12001866; cloning vector: pME18SFL3). This DNA sequence has a length of 3424 nucleotides and carries 4 mutations as compared to SEQ ID NO:1, namely a combination of 54C>T, 76C>T, 219T>C and 1324T>C (numbering corresponding to SEQ ID NO:1).

Although the polynucleotides of the invention may be of various origin including (semi)synthetic origin, it is preferred that the polynucleotide is mRNA or genomic DNA. Standard procedures may be employed to obtain any of the above polynucleotides; see for example, Sambrook et al., "Molecular Cloning, A Laboratory Manual, 2^{nd} ed. 1989, CSH Press, Cold Spring Harbor, N.Y. The polynucleotides of the invention can be prepared by various methods. A full length clone encoding human ERMAP or an allelic variant thereof can be obtained by conventional cloning procedures. Methods for preparing cDNA and genomic clones are well known and within the level of ordinary skill in the art, and include the use of the sequences disclosed herein, or parts thereof, for probing or priming a library. Further polynucleotides of the invention can be obtained by methods such as site directed mutagenesis. The molecules of the invention may also be synthesized by enzymatically linking chemically synthesized fragments.

The invention further relates to a vector comprising a polynucleotide according to the invention. The vector may be used for propagation and/or expression or may be designed for gene transfer or targeting purposes. Methods of producing such vectors are well known in the art. The same holds true for cloning the polynucleotides into said vectors, as well as the propagation of vectors in suitable hosts, etc.

The vector may particularly be a plasmid, a cosmid, a virus or a bacteriophage used conventionally in genetic engineering that comprise a polynucleotide according to the invention. Expression vectors derived from viruses such as retroviruses, vaccinia virus, adeno-associated virus, herpes viruses, or bovine papilloma virus, may be used for delivery of the polynucleotide or vector of the invention into targeted cell populations. Methods which are well known to those skilled in the art can be used to construct recombinant viral vectors; see, for example, the techniques described in Sambrook et al., "Molecular Cloning, A Laboratory Manual, 2^{nd} ed. 1989, CSH Press, Cold Spring Harbor, N.Y. and Ausubel et al., Current Protocols in Molecular Biology, Green Publishing Associates and Wiley Interscience, N.Y. (1989). Alternatively, the polynucleotides and vectors of the invention can be reconstituted into liposomes for delivery to target cells. The vectors containing the polynucleotides of the invention can be transferred into the host cell by well-known techniques, which vary depending on the type of cellular host. For example, calcium chloride transfection is commonly utilized for prokaryotic cells, whereas calcium phosphate treatment or electroporation may be used for other cellular hosts; see Sambrook et al., supra.

Such vectors may comprise further genes such as marker genes which allow for the selection of said vector in a suitable host cell and under suitable conditions. Preferably, the polynucleotides according to the invention is operably linked to expression control sequences allowing expression in prokaryotic or eukaryotic cells. Expression of said polynucleotide comprises transcription of the polynucleotide into a translatable mRNA. Regulatory elements ensuring expression in eukaryotic cells, preferably mammalian cells, are well known to those skilled in the art. They usually comprise regulatory sequences ensuring initiation of transcription and optionally poly-A signals ensuring termination of transcription and stabilization of the transcript. Additional regulatory elements may include transcriptional as well as translational enhancers, and/or naturally-associated or heterologous promoter regions. Possible regulatory elements permitting expression in prokaryotic host cells comprise, e.g., the PL, lac, trp or tac promoter in E.coli, and examples for regulatory elements permitting expression in eukaryotic host cells are the AOX1 or GAL1 promoter in yeast or the CMV-, SV40-, RSV-promoter (Rous sarcoma virus), CMV-enhancer, SV40-enhancer or a globin intron in mammalian and other animal cells. Beside elements which are responsible for the initiation of transcription such regulatory elements may also comprise transcription termination signals, such as the SV40-poly-A site or the tk-poly-A-site, downstream of the coding sequence. Furthermore, depending on the expression system used leader sequences capable of directing the polypeptide to a cellular compartment or secreting it into the medium may be added to the coding sequence of the polynucleotide of the invention and are well known in the art. The leader sequence(s) is (are) assembled in appropriate phase with translation, initiation and termination sequences, and preferably, a leader sequence capable of directing secretion of translated protein, or a portion thereof, into the periplasmic space or extracellular medium. Optionally, the heterologous sequence can encode a fusion protein including a C- or an N-terminal identification peptide imparting desired characteristics, e.g., stabilization or simplified purification of expressed recombinant product. In this context, suitable expression vectors are known in the art such as Okayama-Berg cDNA expression vector pcDV1 (Pharmacia), pCDM8, pRc/CMV, pcDNA1, pcDNA3 (Invitrogen), or pSPORT1 (GIBCO BRL).

Preferably, the expression control sequences will be eukaryotic promoter systems in vectors capable of transforming or transfecting eukaryotic host cells, but control sequences for prokaryotic hosts may also be used.

As mentioned above, the vector of the present invention may also be a gene transfer or targeting vector. Gene therapy, which is based on introducing therapeutic genes into cells by ex vivo or in vivo techniques is one of the most important applications of gene transfer. Suitable vectors and methods for in vitro or in vivo gene therapy are described in the literature and are known to the person skilled in the art; see, for example, Giordano, Nature Medicine 2 (1996), 534-539; Schaper, Circ. Res. 79 (1996), 911-919; Anderson, Science 256 (1992), 808-813; Isner, Lancet 348 (1996), 370-374; Muhlhauser, Circ. Res. 77 (1995), 1077-1086; Wang, Nature Medicine 2 (1996), 714-716; WO94/29469; WO97/00957 or Schaper, Current Opinion in Biotechnology 7 (1996), 635-640, and references cited therein. The polynucleotides and vectors of the invention may be designed for direct introduction or for introduction via liposomes, or viral vectors (e.g. adenoviral, retroviral) into the cell. Preferably, said cell is a germ line cell, embryonic cell, or egg cell or derived therefrom, most preferably said cell is a stem cell.

The polynucleotides of the invention preferably are isolated polynucleotides. The term "isolated", when applied to a polynucleotide, denotes that the polynucleotide has been removed from its natural genetic milieu and is thus free of other extraneous or unwanted coding sequences, and is in a form suitable for use within genetically engineered protein production systems. Such isolated molecules are those that are separated from their natural environment and include cDNA and genomic clones. Isolated DNA molecules of the present invention are free of other genes with which they are ordinarily associated, but may include naturally occurring 5' or 3' untranslated regions such as promoters and terminators. The identification of associated regions will be evident to one of ordinary skill in the art (see for example, Dynan and Tijan, Nature 316 (1985), 774-778).

Additionally, the invention relates to a non-human host transformed or transfected with a vector according to the invention. Suitable hosts comprise non-human transgenic animals, cells such as bacteria, yeast cells, animal, preferably mammalian cells, fungal cells or insect cells. Transformation and transfection protocols including microinjection and electroporation are well known to those skilled in the art.

A further aspect of the invention is a method of producing a Scianna antigen or a fragment thereof comprising culturing a host according to the invention under suitable conditions and isolating the Scianna antigen or fragment thereof. Transformed or transfected host cells are cultured according to conventional procedures in a culture medium containing nutrients and other components required for the growth of the chosen host cell. It is preferred that the antigen is exported into the culture medium where it can be collected according to conventions/methods.

It is preferred to purify the polypeptides or Scianna antigens of the inventions to >80% purity, more preferably to >90% purity, and particularly preferred is a pharmaceutically pure state, that is greater than 99.9% pure with respect to contaminating macromolecules, particularly other proteins and polynucleotides, and free of infectious and pyrogenic agents. Preferably, a purified polypeptide or Scianna antigen is substantially free of other polypeptides, particular other polypeptides of animal origin. Expressed recombinant Scianna antigens can be purified using fractionation and/or conventional purification methods and media. Methods of protein purification are described in e.g., Methods in Enzymology, Vol. 182, "Guide to Protein Purification"; Scopes, "Protein Purification", Springer Verlag, Heidelberg 1994; Deutscher, "Protein Purification", Academic Press, New York 1990.

The invention further relates to a polypeptide encoded by the polynucleotide of the invention and/or obtainable by the above-described method of producing a Scianna antigen or a fragment thereof. Full length human wild type ERMAP (SEQ ID NO:2) is not within the scope of the present invention. In one embodiment, the polypeptide is in the same way posttranslationally modified and has the same chemical structure as the naturally occurring polypeptide. Accordingly, said polypeptide, when produced by the method of the invention, may be produced in human cells.

Preferably, the polypeptide of the invention is an isolated polypeptide. An "isolated" polypeptide or protein is a polypeptide or protein that is found in a condition other than its native environment, such as apart from blood and animal tissue. In a preferred form, the isolated polypeptide is substantially free of other polypeptides, particularly other polypeptides of animal origin. It is preferred to provide the polypeptides in a highly purified form, i.e., greater than 90% pure, more preferably greater than 95% pure, most preferably greater than 99% pure. When used in this context, the term "isolated polypeptide" does not exclude the presence of the same polypeptide in alternative physical forms, such as dimers or alternatively glycosylated or derivatized forms.

The polypeptides of the invention may be prepared according to a method of the invention described above. They may also be prepared by chemical synthesis, e.g. solid phase synthesis. The latter method is particularly preferred when producing shorter polypeptides having a length of up to 50 amino acids, preferably up to 35 amino acids, most preferably up to 20 amino acids.

A further aspect of the invention is an oligonucleotide capable of hybridising under stringent conditions to a portion of the polynucleotide of the invention comprising said at least one mutation or to the complementary portion thereof. In this embodiment, it is understood that the oligonucleotide hybridizes directly to the mutated sequence. The setting of stringent conditions is well described, for example, in Sambrook et al., "Molecular Cloning, A Laboratory Manual, 2^{nd} ed. 1989, CSH Press, Cold Spring Harbor, N.Y. and Ausubel et al., Current Protocols in Molecular Biology, Green Publishing Associates and Wiley Interscience, N.Y. (1989), or Hames and Higgins, "Nucleic acid hybridization, a practical approach", IRL press, Oxford (1985). Thus, the detection of the specifically hybridizing sequences will usually require hybridization and washing conditions such as 0.1xSSC, 0.1% SDS, 65°C. As is well known, the length of the probe and the composition of the polynucleotide to be determined constitute further parameters of the stringent hybridization conditions. Preferably, the oligonucleotide is a deoxyoligonucleotide. It is further preferred that the oligonucleotide has a length of 12 to 50 nucleotides, more preferably of 15 to 24 nucleotides.

If the oligonucleotide is to be used as a primer, it is preferred that the nucleotide in the oligonucleotide detecting the presence or absence of a mutation, i.e. conferring the specificity, is the 3'-most nucleotide of the oligonucleotide. Examples of such oligonucleotides are oligonucleotides having a sequence selected from the group consisting of SEQ ID NO:12, SEQ ID NO:13, SEQ ID NO:14, SEQ ID NO:15, SEQ ID NO:16, SEQ ID NO:17 and SEQ ID NO:18.

In one embodiment, the oligonucleotide does not hybridize under stringent conditions to the wild type allele, i.e. to the polynucleotide as shown in SEQ ID NO:1.

In another aspect, the invention relates to an oligonucleotide specifically hybridizing under stringent conditions to a portion of the wild type allele sequence but not to a polynucleotide of the invention corresponding to the said portion of the wild type allele.

The invention further relates to an antibody or aptamer or phage specifically binding to a polypeptide of the invention. In one embodiment, the antibody or aptamer or phage does not bind to to human wild type ERMAP. The antibody may be tested and used in any serologic technique well known in the art, like agglutination techniques in tubes, gels, solid phase and capture techniques with or without secondary antibodies, or in flow cytometry with or without immunofluorescence enhancement. The antibody of the invention may be a monoclonal antibody or an antibody derived from or comprised in a polyclonal antiserum. The term "antibody", as used herein, further comprises fragments of said antibody such as Fab, F(ab')₂, Fv or scFv fragments: see, for example Harlow and Lane, "Antibodies, A Laboratory Manual" CSH Press 1988, Cold Spring Harbor N.Y. The antibody or the fragment thereof may be of natural origin or may be (semi)synthetically produced. Such synthetic products also comprises non-proteinaceous or semi-proteinaceous material that has the same or essentially the same binding specificity as the antibody of the invention. Such products may, for example be obtained by peptidomimetics. Examples of preferred antibodies are antibodies specifically recognizing the presence or the absence of the H26Y amino acid substitution, of the G57R amino acid substitution or of the P60A amino acid substitution.

Another aspect of the invention is an antibody, aptamer or phage specifically binding to wild type ERMAP but not to a polypeptide of the invention. As regards the definition, testing and origin of the antibody, the same definitions as described supra apply here.

Preferably, the antibody of the invention is an isolated antibody. The definition of "isolated polypeptide" applies to antibodies as well. It is to be understood, that the term "isolated polyclonal antibody" does not exclude the presence of a plurality of isotypes having the same specificity. The antibodies of the invention can be used in the methods described throughout the present specification.

The term "aptamer" is well known in the art and defined, e.g., in Osborne et al. Curr. Opin. Biol. 1 (1997), 5-9 or in Stall and Szoka, Pharm. Res. 12 (1995), 465-483.

The invention further relates to a method for testing for the presence of a polynucleotide encoding a Scianna antigen in a sample comprising detecting the presence or absence of at least one mutation in a polynucleotide representing the ERMAP gene. Preferably, the method comprises hybidizing an oligonucleotide of the invention under stringent conditions to polynucleotides contained in the sample obtained from a human and detecting said hybridization. Preferably, the method further comprises digesting the product of said hybridization with a restriction enzyme and analyzing the product(s) of said digestion.

This preferred embodiment of the invention allows by convenient means, the differentiation between an effective hybridization and a non-effective hybridization. For example, if the gene encoding ERMAP comprises an endonuclease restriction site, the hybridized product will be cleavable by an appropriate restriction enzyme whereas a mutated sequence will yield no double-stranded product or will not comprise the recognizable restriction site and, accordingly, will not be cleaved. Altematively, the hybridizing oligonucleotide may only hybridize to the mutated sequence. In this case, only a hybrid comprising the mutated sequence, but not the wild type sequence, will be cleaved by the appropriate restriction enzyme. The analysis of the digestion product can be effected by conventional means, such as by gel electrophoresis which may be optionally combined by the staining of the nucleic acid with, for example, ethidium bromide. Combinations with further techniques such as Southern blotting are also envisaged.

Detection of said hybridization may be effected, for example, by an anti-DNA double-strand antibody or by employing a labeled oligonucleotide. Conveniently, the method of the invention is employed together with blotting techniques such as Southem or Northern blotting and related techniques. Labeling may be effected, for example, by standard protocols and includes labeling with radioactive markers, fluorescent, phosphorescent, chemiluminescent, enzymatic labels, etc.

In another embodiment, the method comprises determining the nucleic acid sequence of at least a portion of the polynucleotide representing the ERMAP gene. Preferably, prior to determining said sequence of the polynucleotide, amplification of at least said portion of the polynucleotide is performed. Preferably, amplification is effected by polymerase chain reaction (PCR). Other amplification methods such as ligase chain reaction may also be employed.

In yet another embodiment, the method comprises carrying out an amplification reaction wherein at least one of the primers employed in said amplification reaction is an oligonucleotide of the invention.

The method of the invention will result in an amplification of only the target sequence, if said target sequence carries the or at least one mutation. This is because the oligonucleotide will under preferably stringent hybridization conditions not hybridize to the wild type sequence (with the consequence that no amplification product is obtained) but only to the mutated sequence. Naturally, primer oligonucleotides hybridizing to one or more as one, such as two mutated sequences may be employed in the method of the invention. The latter embodiment may be favorable in cases where combinations of mutations are tested for. It is important to note that not all or none of said mutations are necessarily missense mutations.

Preferably, in the method of the invention said amplification or amplification reaction is or is effected by the polymerase chain reaction (PCR). Other amplification methods such as ligase chain reaction may also be employed.

The invention further relates to a method for testing for the presence of a Scianna antigen in a sample comprising assaying a sample obtained from a human for specific binding to an antibody or aptamer or phage of the invention. The testing for binding may involve the employment of standard techniques such as ELISAs; see for example, Harlow and Lane, "Antibodies, A laboratory Manual, CSH Press 1988, Cold Spring Harbor. Results obtained in accordance with the method of the invention may well be employed in strategies of blood transfusion, as outlined herein. In particular, the methods of the invention are useful in determining the allelic status of an individual and in blood group determination.

Preferably, in the methods of the invention, the sample obtained from a human is blood, serum, plasma, fetal tissue, saliva, urine, mucosal tissue, mucus, vaginal tissue, fetal tissue obtained from the vagina, skin, hair, hair follicle or another human tissue.

In a particular embodiment of the invention, the polynucleotide material or proteinaceous material from said sample is fixed to a solid support. Preferably, the solid support is a chip. The chip technology is known to those of ordinary skill (McGlennen, Clin Chem 47(3), 393-402 (2001)). The advantages of chips include the small size as well as an easy access of computer based analysis of analytes.

Another aspect of the invention is the use of a polynucleotide of the invention, or of an oligonucleotide of the invention, or of an antibody or aptamer or phage of the invention for the detection of a Scianna antigen and/or determination of the Scianna antigen type. The preferred embodiments of such use have been outlined above with respect to the method of the invention. The detection and/or determination can be effected on the basis of the methods described herein.

The invention also relates to the use of the polynucleotide of the invention, the vector of the invention or the polypeptide of the invention for the assessment of the affinity, avidity and/or reactivity of monoclonal anti-Sc antibodies or of polyclonal anti-Sc antisera or of anti-globulin or of anti-human-globulin antisera or of preparations thereof. As used herein, the term "anti-Sc" (antibodies, antisera, etc.) comprises anti-Scianna and anti-Radin (antibodies, antisera, etc.).

The invention also relates to the use of cells, preferably red blood cells, from probands for serologic tests, e. g. for the assessment of the affinity, avidity sensitivity, specificity and/or reactivity of monoclonal anti-Sc antibodies or of polyclonal anti-Sc antisera or of anti-globulin or of anti-human-globulin antisera or of preparations thereof. The cells preferably have been characterized by a method of the invention before their use in a serologic test.

Said preparations can be provided according to techniques well known in the art. Said preparations may comprise stabilisators such as albumins, further sodium azide, salt ions, buffers etc. The formulation of the preparation may have an influence on the binding characteristics of the antibodies, as is well known in the art.

For example, in a first step, the ERMAP gene of a carrier or of a blood donor and its allelic status is analyzed and it is determined whether said gene comprises a mutation that was found in accordance with the present invention. In a second step, said mutation is correlated to the presence of a certain Scianna (Sc) antigen on the surface of red blood cells. Conveniently, said correlation can be established by data provided in the present invention (such as mutations per se) and techniques that are well known in the art (see, e.g. Jones et al. 1996, Vox Sanguinis 71:176-183; Flegel and Wagner, 1996, Transfusion Clinique et Biologique 3:429-431). In a third step, the features of an antibody or an antiserum such as reactivity, sensitivity, affinity, avidity, and/or specificity are determined with suitable blood group serological techniques preferably using red blood cells that were molecularly and with respect to the Sc antigen status characterized as described in step 2. Such data can be used, for example, in quality controls, standardization, etc.

The invention will be most useful for the characterization, standardization and quality control of monoclonal and polyclonal antisera, preferably anti-Sc monoclonals or antisera. Further, for example, anti-globulin and anti-human-globulin antisera can be characterized on the basis of the teachings of the present invention. An appropriately characterized anti-Sc monoclonal antibody can be conveniently used in Scianna diagnostics. For example, a suitably characterized monoclonal antibody will be useful in determining the presence or absence of a certain Sc antigen on the surface of blood cells. Cut-off values for monoclonal antibodies useful in diagnosis can thus be established. This is important for the quality control of antibodies used in Scianna diagnosis.

Thus, the invention also relates to a method for the characterization of monoclonal antibodies or polyclonal antisera or of a preparation thereof, said method comprising
(a) testing the polynucleotide of sample of a proband for the presence of a mutation as defined in accordance with the invention;
(b) correlating, on the basis of the mutation status and the allelic status of the ERMAP gene, the polynucleotide with the presence of a certain Sc antigen on the surface of red blood cells of said proband;
(c) reacting said monoclonal antibodies or polyclonal antisera or said preparation thereof with a cell carrying the said Sc antigen on its surface;
(d) characterizing said monoclonal antibodies or polyclonal antisera or said preparation thereof on the basis of the results obtain in step (c).

As regards the term "allelic status", this term describes the possibilities that the ERMAP alleles in a proband are present in a homozygous, heterozygous or hemizygous state. Also comprised by this term is the possibility that the two alleles carry two different mutations defined herein above.

In a preferred embodiment of the method of the invention, said characterization comprises the determination of reactivity, sensitivity, avidity, affinity, specificity and/or other characteristics of antibodies and antisera.

Furthermore preferred is a method wherein said cell carrying a certain Sc antigen on its surface is a red blood cell.

The invention also relates to a method for determining whether a patient in need of a blood transfusion is to be transfused with blood from a donor having a certain Sc status. The method comprises the step of testing a sample from said patient for the presence of one or more Sc antigens of the invention, wherein a negative testing for at least one of said antigens is indicative of the need for a transfusion with blood which is negative for the said Sc antigen. The invention has important implications for devising a transfusion therapy in humans. For example, it can now be conveniently tested whether the patient actually needs a transfusion with a blood negative for a certain Sc antigen or whether such precautions need not be taken.

Furthermore, the invention relates to a method for determining whether blood of a donor may be used for transfusion to a patient in need thereof comprising the step of testing a sample from said donor for the presence of one or more Sc antigens of the invention, wherein a positive testing for at least one of said antigens excludes the transfusion of patients that are typed as not having said Sc antigen.

The samples referred to in the above recited methods may be samples that are referred to throughout the specification, such as blood, serum, etc.

As regards the guidelines for transfusing a patient on the basis of any of the above recited methods, the utmost care must be taken that suboptimal transfusion policy is avoided. The risk factor is always to be considered by the physician in charge. In all cases. the potential risk for the patient is to be minimized.

The invention also relates to the use of a phage, aptamer, monoclonal antibody or a polyclonal antisera or a preparation thereof as characterized in the present invention for Sc antigen determination. In a preferred embodiment of said use, said Sc antigen determination is effected in connection with blood group typing.

Furthermore, the invention relates to a preparation comprising the antibody or aptamer or phage of the invention.

Antibodies and preparations thereof may be tested by any standard blood group serology technique with one or more Sc types of the invention. The reactivity, sensitivity, avidity, affinity, specificity and/or other characteristics of antibodies and antisera known in the art may be determined by its reaction with one or more Sc antigen types of the invention under predetermined conditions in standard blood group serology techniques well know in the art. The preparation may be a diagnostic or pharmaceutical preparation.

The pharmaceutical composition of the present invention may further comprise a pharmaceutically acceptable carrier and/or diluent. Examples of suitable pharmaceutical carriers are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical, intradermal, intranasal or intrabronchial administration. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. A typical dose can be, for example, in the range of 0.001 to 1000 µg; however, doses below or above this exemplary range are envisioned, especially considering the aforementioned factors. Generally, the regimen as a regular administration of the pharmaceutical composition should be in the range of 1 µg to 10 mg units per day. If the regimen is a continuous infusion, it should also be in the range of 1 µg to 10 mg units per kilogram of body weight per minute, respectively. Progress can be monitored by periodic assessment. The compositions of the invention may be administered locally or systemically.

Administration will generally be parenterally, e.g., intravenously; DNA may also be administered directly to the target site; e.g., by biolistic delivery to an internal or external target site or by catheter to a site in an artery. Preparations for parenteral administration include sterile aqueous or non-aqueous solutions, suspensions, and emulsions. Examples of non-aqueous solvents are propylene glycol, polyethylene glycol. vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. Furthermore, the pharmaceutical composition of the invention may comprise further agents such as interleukins or interferons depending on the intended use of the pharmaceutical composition.

An antibody and its preparation may be characterized by its reaction or lack of reaction to surfaces with certain Sc antigens. For example, antibody preparations may be characterized by agglutinating red blood cells being Sc+1, Sc+2 and/or Rd+.

The invention also relates to treating a pregnant woman being Sc-1 or Sc-2 or being hemizygous for a mutation defined herein above wherein the child is Sc+1 or Sc+2, respectively, or carries a different mutation defined herein above in a hemizygous state comprising administering the respective anti-Sc to said woman.

The administration can be effected by standard routes and doses which can be defined by the attending physician: Mollison, 1993. Preferably, a monoclonal anti-Sc or combinations/mixtures of monoclonal anti-Scs is/are administered in doses of 50 µg to or exceeding 500 µg anti-Sc antibody/antisera for intravenous or intramuscular administration (Bowman, 1998). For the quality control of these anti-Sc antibodies/antisera, the results and methods provided by the present invention may be advantageously employed.

The present invention also relates to a method of identifying an antibody V_{H} or V_{L} chain or a combination thereof or an aptamer specifically binding to a Sc polypeptide of the invention comprising
(a) contacting the Sc polypeptide of the invention with a phage library displaying V_{H} or V_{L} chains or combinations thereof on the surface of the phage or with aptamers;
(b) identifying phage or aptamers that bind to said Sc polypeptide; and optionally
(c) repeating steps (a) and (b) one or more times.

The preparation of phage library and the screening/identification of desired antibody (chains) per se is well known in the art and reviewed, for example, in Winter et al., Annu. Rev. Immunol. 12 (1994), 433-455 and references cited therein. Also, aptamers can be prepared and cloned in phage according to conventional protocols. Whereas single V_{H} or V_{L} chains may be identified by the method of the invention as binding to the Sc polypeptide of the invention, it is preferred to identify V_{H}-V_{L} combinations expressed by the phage because this situation resembles the situation of natural antibody binding. By repeating steps (a) and (b) one or more times, better binding specificities may be identified. Protocols for the optimization of binding properties such as affinities, including elution steps for removing bound phage, are well established in the art. For example, once a V_{H} chain with a convenient binding capacity has been found, V_{L} chains may be identified that significantly improve the binding capacity of the antibody, e.g. by replacing the V_{L} chain that was associated with the V_{H} chain in the first selection step with a more suitable V_{L} chain.

The invention also relates to a method of identifying a monoclonal antibody specifically binding to a Sc polypeptide/antigen of the invention comprising
(a) contacting the Sc polypeptide of the invention with one or more monoclonal antibodies;
(b) identifying monoclonal antibodies that bind to said Sc polypeptide; and optionally
(c) repeating steps (a) and (b) one or more times.

Preferably, the Sc polypeptide is exposed on the surface of a cell. An appropriate surface is the surface of an erythrocyte. However, other host cells may be transfected with a vector suitable for expression of the Sc polypeptide and express the same on their surface. Antibodies may also bind to recombinant proteins of or parts of proteins of Sc and purified proteins.

It is further preferred that the polypeptide or host cell is affixed to a solid support. Suitable examples for solid supports are microtiter plates or beads.

The invention further relates to a kit which is useful in the methods described herein. The kit comprises at least one oligonucleotide of the invention and/or at least one antibody or aptamer or phage of the invention. The kit may comprise various types of antibodies described herein above, and is particularly suitable for the analysis of the Scianna antigen type in samples obtained from humans. The components of the kit may be packaged as appropriate. Preferably, different components are packaged in different vials. The components may be provided in a container.

The antigens of the Scianna blood group system are carried by the ERMAP protein. It has formally been proven by the inventors that the low frequency antigen Radin (Rd) belongs to the Scianna blood group system, because it is expressed by a variant ERMAP protein. A third amino acid polymorphism H26Y was frequently observed but could not be related to a known blood group antigen.

The identity of the *ERMAP* gene with the gene underlying the Scianna blood group system was based on two independent lines of evidence: (i) The expression of the Sc antigens was lacking in a proband carrying a nonfunctional *ERMAP* allele in homozygous form. (ii) The expression of the Sc1 and Sc2 antigens correlated with the presence of a wild type *ERMAP* allele and the otherwise rare *ERMAP(G57R)* allele, respectively. Each observation alone could have been explained by a linkage to a neighboring gene, their coincidence, however, must be considered proof of the identity of ERMAP and Scianna.

The low frequency antigen Radin (Rd) was suspected to belong to the Scianna blood group (Spring, 1997), but a final verification was lacking. We showed Rd to be associated with the distinct rare *ERMAP(P60A)* allele, rendering Rd part of the Scianna blood group system. Sc:-1,-2 RBCs have been reported to lack several distinguishable high-incidence antigens (Devine et al., 1988). It will be straightforward to check for the association of high and low frequency antigens with *ERMAP* alleles, possibly extending the number of antigens known to belong to this blood group system.

*ERMAP* is the fifth blood group protein being member of to the immunoglobulin superfamily, along with LU (ISBT 005) (Parsons et al., 1995), LW (ISBT 016) (Bailly et al., 1994), and OK (ISBT 024) (Spring et al., 1997). As expected, the amino acids involved in the Sc1/Sc2 and Rd blood group polymorphisms occurred in an extracellular protein segment. The glycine at position 57 representative of the Sc1 antigen was likely located beneath the protein's surface, as shown by modeling of the immunoglobuline domain of ERMAP. However, the amino acid substitution in Sc2 was predicted to cause considerable displacement of the neighboring amino acids, which may explain the immunogenicity.

A third amino acid polymorphism Y26H was frequent in European population. Because the 29 aminoterminal amino acids of ERMAP probably represented its signal peptide, this polymorphism was not expected to be part of the mature ERMAP protein. Accordingly, a correlation with a blood group antigen was neither expected nor established.

Scianna was the last previously defined blood group system encoded by a protein whose molecular basis was resolved. Hence, the identification of the gene underlying Scianna rendered genotyping possible for all protein encoded blood groups. The ERMAP protein is believed to represent an adhesion protein and possesses a C1q recognition sequence (Xu et al., 2001; Su et al., 2001). However, its function has not been fully established. Since the probands of the known kindreds with Sc:-1,-2 phenotype appear to be healthy (lssitt and Anstee, 1998), its function likely may be compensated by other proteins. Even the lack of functional important proteins as the water transporter in Co(a-b-) individuals (Joshi et al., 2001) may become symptomatic only in very special circumstances (King et al., 2001). It may be worthwhile to check if the polymorphisms observed in *ERMAP* influence its binding characteristics and function.

Figure 1 shows a two base pair (bp) deletion in the *ERMAP* allele of the Sc:-1,-2 proband. The nucleotide sequences (nt) and derived amino acid sequences (aa) of a part of the *ERMAP* exon 3 are shown for the proband with the Sc:-1,-2 phenotype (panel A) and a control with the prevalent *ERMAP* allele encoding Sc:1,-2 (panel B). The control harbors a triple "GA"-repeat at nucleotides 303 to 308, one of which is deleted in the Sc:-1,-2 proband (asterisks). Because of the induced frame-shift, a non-sense amino acid sequence is translated beyond codon 102 and a stop codon occurs at codon 114, resulting in a non-functional protein of 113 amino acids. All nucleotides are in capitals indicating an exon sequence.

**Figure 2** shows Scianna genotyping by PCR-SSP. PCR were devised to amplify specific PCR products of 147 bp size from the wild type *ERMAP* allele representative of Sc1 (Panel A) or from the *ERMAP(G57R)* allele carrying the Sc2 antigen (Panel B), respectively. Representative results for Sc:1,-2; Sc:1,2; Sc:-1,2 and Sc:-1,-2 samples are shown. Control PCR products of 434 bp size occurred in all reactions.

**Figure 3** shows Radin genotyping by PCR-SSP. A PCR was devised to amplify a specific PCR product of 156 bp size from the *ERMAP(P60A)* allele carrying the Rd antigen. Representative results for Sc:1,-2; Sc:1,2; Sc:-1,2 and Sc:-1,-2 samples and an Rd+ sample are shown. The 434 bp control product derives from the *HGH* gene but this product may be lacking because of competition, if a specific product is present.

**Figure 4** shows the nucleotide sequence and the deduced amino acid sequence of human wild type ERMAP.

**Figure 5** shows the nucleotide sequence of an ERMAP allele carrying a 76C>T mutation and the deduced amino acid sequence carrying an H26Y amino acid subsitution. This allele represents a molecular basis of the Sc +1 phenotype.

**Figure 6** shows the nucleotide sequence of an ERMAP allele carrying a 76C>T and a 54C>T mutation and the deduced amino acid sequence carrying an H26Y amino acid subsitution. This allele represents a molecular basis of the Sc +1 phenotype.

**Figure 7** shows the nucleotide sequence of an ERMAP allele carrying a 169G>A mutation and the deduced amino acid sequence carrying an G57R amino acid subsitution. This allele represents the molecular basis of the Sc +2 phenotype.

**Figure 8** shows the nucleotide sequence of an ERMAP allele carrying a 76C>T, a 54C>T mutation and a 307del2 deletion and the deduced amino acid sequence carrying an H26Y amino acid subsitution and a frameshift resulting in a truncated protein. This allele represents a molecular basis of the Sc -1-2 phenotype.

**Figure 9** shows the nucleotide sequence of an ERMAP allele carrying a 178C>G mutation and the deduced amino acid sequence carrying an P60A amino acid subsitution. This allele represents the molecular basis of the Rd+ phenotype.

The following example further illustrates the invention.

### 1. Subjects and Methods

**Rare Scianna phenotypes.** Sc:+2 and Rd+ blood samples were obtained from the SCARF exchange program. Sc:-1,+2 and Sc:-1,-2 samples were referred to the International Blood Group Reference Laboratory. The Sc:-1,-2 pedigree came from Saudi-Arabia. Control blood samples derived from South-Western German blood donors.

**Nucleotide sequencing.** Nucleotide sequencing was performed using Prism dye terminator cycle-sequencing kit with AmpliTaq FS DNA polymerase and Prism BigDye terminator cycle sequencing ready reaction kit (Applied Biosystems, Weiterstadt, Germany) with a DNA sequencing unit (ABI 3100, Applied Biosystems). Nucleotide sequencing of genomic DNA stretches representative for all eleven *ERMAP* exons and parts of the promoter was accomplished using primers (Table 1) and amplification procedures (Table 2) that obviated the need for subcloning steps. PCR was performed using Expand high-fidelity PCR system or Expand long range PCR system (Boehringer Mannheim, Mannheim, Germany).

**PCR-SSP.** PCR-SSP methods were devised to detect the C/T polymorphism at nucleotide 54 (counted relative to the A of the start codon in the cDNA), the C/T polymorphism at position 76, the G/A polymorphism at position 169 causing the Sc1/Sc2 polymorphism and the G at position 178 in Rd (Table 3). The reactions were triggered to work under similar PCR conditions as a PCR-SSP system previously developed for *RHD* typing (Gassner et al., 1997; Wagner et al., 2001). Amplifications were carried out with Taq (Qiagen, Hilden, Germany) in a final volume of 10 µl. *HGH* control primers were used at a concentration of 0.75 µM to amplify the 434-bp control PCR fragment derived from the *human growth hormone* gene.

**PCR-RFLP.** The PCR product also used for exon 3 sequencing (Table 2) was digested for 3 h at 37°C with *Pvu*ll and *Sma*l (Pharmacia, Freiburg, Germany).

**Population screen for *ERMAP* alleles.** The prevalence of the 54 T/C and 76 T/C polymorphism were determined in 111 blood samples from blood donors in Baden-Württemberg (South-Western Germany) by PCR-SSP. Maximum-likelihood estimates of allele frequencies were calculated using the counting method (Ceppellini et al., 1955). The presence of *ERMAP* alleles representative of Sc2 and Rd was checked by PCR-SSP in 305 samples.

**Modeling.** The extent of the signal peptide was determined using Signal P V2.0 (http://www.cbs.dtu.dk/services/SignalP-2.0/) web server (Nielsen et al., 1997; Nielsen and Krogh, 1998) . A three-dimensional model of the immunoglobulin domain of ERMAP was modeled at the 3D-JIGSAW server (http://www.bmm.icnet.uk/∼3djigsaw/) (Bates et al., 2001; Bates et al., 1999) using the heavy chain of the Fab fragment of the murine monoclonal antibody 3A2 (Fotinou et al., 1998) as template.

### 2. Results

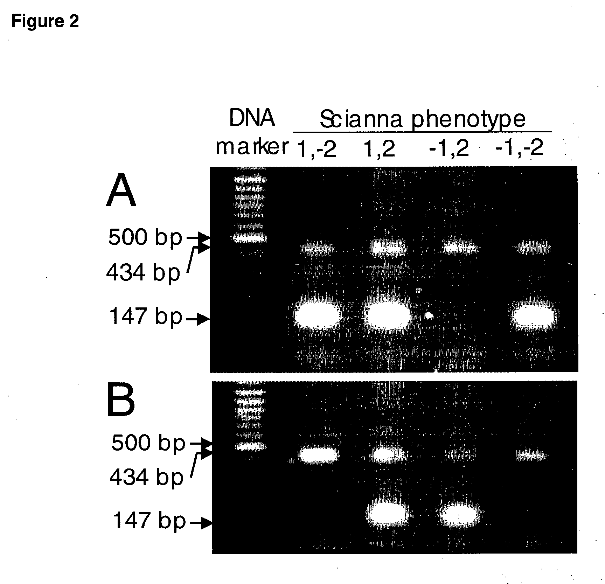

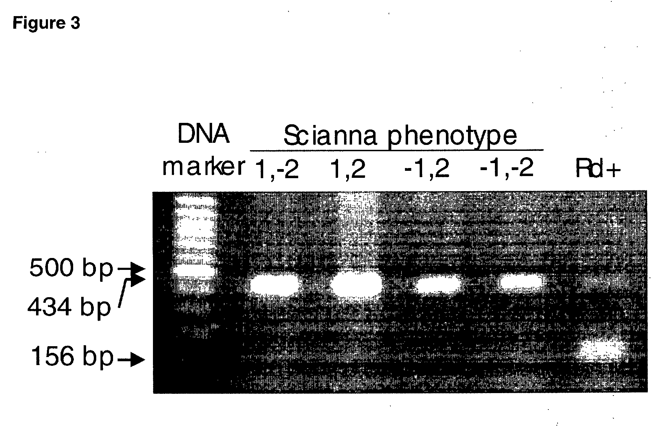

The chromosomal position of the *ERMAP* gene and the molecular weight of the encoded erythroid membrane-associated protein (ERMAP) matched the data closely that were previously established for the Scianna glycoprotein (Xu et al., 2001; Su et al., 2001). These observations prompted us to explore whether *ERMAP* alleles were underlying the Scianna antigens.
**a) A Sc:-1-2 proband is homozygous for a non-functional *ERMAP* allele.**
   The nucleotide sequence of the 11 *ERMAP* exons in a proband who lacked both the Sc1 and Sc2 antigens was determined. The *ERMAP* allele harbored a two base pair deletion in exon 3. This frameshift caused a truncated protein of 113 amino acids (Fig. 1). Additional nucleotide substitutions 54C>T and 76C>T were detected in exon 2, the latter of which caused a histidine to tyrosine amino acid substitution at codon 26 (not shown). No evidence for a second *ERMAP* allele was observed suggesting homozygosity for the ERMAP(54C>T,76C>T,307de12) allele.
**b) The Sc2 antigen is due to an *ERMAP*(G57R) allele.**
   **(i) Sc:+2 samples carry an *ERMAP*(G57R) allele.** The *ERMAP* gene of a Sc:-1,+2 proband was sequenced to determine the molecular basis of the Sc2 antigen and to corroborate that Scianna and *ERMAP* are identical. An adenosine (A) was detected at the predicted cDNA position 169 (relative to the A of the start codon) that indicated the homozygous presence of an *ERMAP* allele with a glycine to arginine substitution in codon 57. The nucleotide substitution destroyed a *Sma*l site and allowed an independent confirmation by PCR-RFLP. The homozygous presence of the *ERMAP*(G57R) allele was demonstrated by nucleotide sequencing of *ERMAP* exon 3 in two additional unrelated Sc:-1,+2 probands. Two Sc:+1,+2 samples were heterozygous for the *ERMAP*(G57R) allele. PCR-SSP methods for the rapid detection of the *ERMAP*(G57R) and the wild type *ERMAP* alleles were developed (Fig. 2).
   **(ii) *ERMAP*(G57R) is rare in the population.** DNA from 305 blood donors was checked for the presence of an *ERMAP*(G57R) allele by PCR-SSP. 5 samples were positive for this allele, which corresponded to a haplotype frequency of 0.008, since the 5 samples carried the wild type *ERMAP* allele, too.
   **(iii) *ERMAP*(G57R) positive samples are Sc:+2.** Red cells of two *ERMAP*(G57R) positive donors detected by PCR-SSP were tested for the Sc1 and Sc2 antigens by serology. Both donors were found to be Sc:+1,+2.
**c) The Radin (Rd) antigen is due to an *ERMAP*(P60A) allele.**
   **(i) An Rd+ sample carries an *ERMAP*(P60A) allele.** The *ERMAP* gene of an Rd+ proband was sequenced to assess if Rd belongs to the Scianna blood group system. A C/G heterozygosity was detected at the predicted cDNA position 178 that indicated the heterozygous presence of an *ERMAP* allele with a proline to alanine substitution at codon 60. A PCR-SSP for the rapid detection of the *ERMAP*(P60A) allele was developed (Fig. 3).
   **(ii) *ERMAP*(P6QA) is very rare in the population.** Among the 305 blood donors previously tested for *ERMAP*(G57R), no donor was positive for the *ERMAP*(P60A) allele. However, among a different set of 20 random donors an *ERMAP*(P60A) positive sample was inadvertently found.
   **(iii) The second *ERMAP*(P60A) positive sample is Rd+.** Red cells of the *ERMAP*(P60A) positive donor detected by PCR-SSP were tested for the Sc1, Sc2, and Rd antigens by serology. He was found to be Sc:+1,-2; Rd+.
**d) Population frequencies of the *ERMAP*(H26Y) allele and the silent 54C>T polymorphism.**
   The 54C>T and 76C>T polymorphism initially observed in the rare Sc:-1,-2 phenotype were also encountered in control samples. Therefore, 111 blood donors were systematically checked for the presence of an *ERMAP(H26Y)* allele or a 54C>T polymorphism (Table 4). Maximum-likelihood estimates of the allele frequencies were derived. *ERMAP(H26Y)* occurred with a frequency of 0.33. The majority of these alleles (allele frequency 0.28) were predicted to carry a 54C>T substitution, too, while an *ERMAP(H26*) with the wild type C at position 54 occurred less frequently (allele frequency 0.05). In contrast, the *ERMAP* alleles encoding a histidine at position 26 carried a C at position 54 (allele frequency 0.67) and represented the wild type allele (Xu et al., 2001; Su et al., 2001).
**e) Modeling of the ERMAP immunoglobulin domain.**
   Based on the known similarity of *ERMAP* to immunoglobulin, we modeled the tertiary structure of the amino acids 46 to 131 representing part of the extracellular domain of *ERMAP.* The proline substituted in the Rd allele was exposed on the protein's surface. The amino acid causing the Sc1/Sc2 polymorphism was unexpectedly hidden beneath the protein's surface thereby rendering it hardly accessible to antibodies. However, the amino acids involved in the Sc2 polymorphism were predicted to alter the three-dimensional structure of this extracellular domain considerably, which may explain the immunogenicity of the *ERMAP* polymorphism.

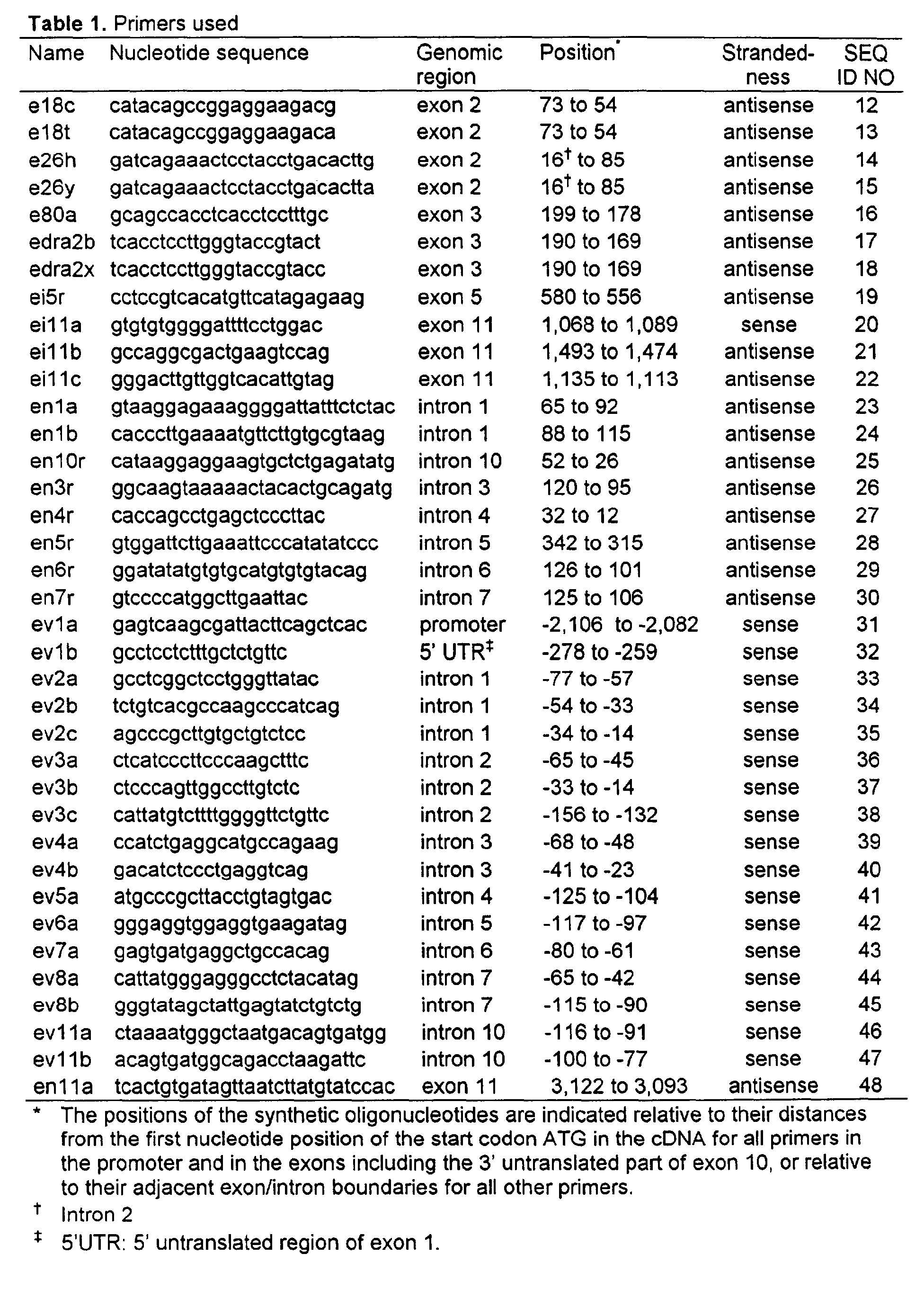

**Table 2.**

| Sequencing method for the 11 *ERMAP* exons from genomic DNA | | | | |
|---|---|---|---|---|
| | PCR primers* | | Sequencing primers*^{†} | |
| *ERMAP* | Sense | Antisense | Sense | Antisense |
| Exon 1 | ev1a | en1b | ev1b | en1b |
| Exon 2 | ev2a | ei5r | ev2b, ev2c | |
| Exon 3 | ev2a | ei5r | ev3c, ev3a | en3r |
| Exon 4 | ev2a | ei5r | ev4a, ev4b | |
| Exon 5 | ev4a | en10r | ev5a | |
| Exon 6 | ev4a | en10r | ev6a | |
| Exon 7 | ev4a | en10r | ev7a | |
| Exon 8 | ev4a | en10r | ev8a | |
| Exon 9 | ev4a | en10r | ev8a | |
| Exon 10 | ev4a | en10r | ev8a | |
| Exon 11 | ev11a | en11a | ev11b, ei11a | ei11b, ei11c |

| | | | | |
|---|---|---|---|---|
| * Primer sequences are given in Table 1. | | | | |
| † For each sample, one or more sequencing primers were used, if applicable. | | | | |

**Table 3.**

| PCR-SSP for *ERMAP* alleles | | | | |
|---|---|---|---|---|
| Polymorphism detected | Detection primers | | | Size of specific |
| | Specific | Non-specific | Concentration | product |
| 54 C | e18c | ev2b | 2 µM | 134 bp |
| 54 T | e18t | ev2b | 2 µM | 134 bp |
| 76 C | e26h | ev2b | 3 µM | 167 bp |
| 76 T | e26y | ev2b | 3 µM | 167 bp |
| 169 A (Sc2) | edra2b | ev3b | 4 µM | 147 bp |
| 169 G (Sc1) | edra2x | ev3b | 4 µM | 147 bp |
| 178 G (Rd) | e80a | ev3b | 2 µM | 156 bp |

**Table 4.**

| Linkage of 54T and 76T polymorphism in 111 samples investigated* | | | | |
|---|---|---|---|---|
| | Position 76 | | | |
| Position 54 | C | C/T | T | Total |
| C | 51 | 7 | 0 | 58 |
| C/T | 0 | 40 | 3 | 43 |
| T | 0 | 0 | 10 | 10 |
| Total | 51 | 47 | 13 | 111 |

| | | | | |
|---|---|---|---|---|
| * Maximum-likelihood estimates of allele frequencies were 0.67 for *ERMAP(54C,76C),* 0.28 for *ERMAP(54T,76T),* 0.05 for *ERMAP(54C, 76T)* and 0 for *ERMAP(54T,76C).* | | | | |

### References

- 1.: Cartron JP, Colin Y. Structural and functional diversity of blood group antigens.
Transfus Clin Biol. 2001;8:163-199.
- 2.: Daniels GL, Anstee DJ, Cartron JP, et al. International Society of Blood Transfusion Working Party on Terminology for Red Cell Surface Antigens. Vox Sang. 2001;80:193-197.
- 3.: Gubin AN, Njoroge JM, Wojda U, et al. Identification of the Dombrock blood group glycoprotein as a polymorphic member of the ADP-ribosyltransferase gene family. Blood. 2000;96:2621-2627.
- 4.: Lewis M, Kaita H, Chown B. Scianna blood group system. Vox Sang. 1974;27:261-264.
- 5.: Issitt PD, Anstee DJ. Applied Blood Group Serology. Miami: Montgomery Scientific Publications; 1998.
- 6.: Spring FA, Herron R, Rowe G. An erythrocyte glycoprotein of apparent Mr 60,000 expresses the Sc1 and Sc2 antigens. Vox Sang. 1990;58:122-125.
- 7.: Lewis M, Kaita H, Chown B. Genetic linkage between the human blood group loci Rh and Sc (Scianna). Am J Hum Genet. 1976;28:619-620.
- 8.: Reid ME, Lomas-Francis C. The Blood Group Antigen Facts Book. San Diego: Academic Press; 1997.
- 9.: Lewis M, Kaita H, Philipps S, et al. The position of the Radin blood group locus in relation to other chromosome I loci. Ann Hum Genet. 1980;44:179-184.
- 10.: Hilden J-O, Shaw M-A, Whitehouse DB, Monteiro M, Tippett P. Linkage information from nine more Radin families [abstract]. Cytogenet Cell Genet. 1985;40:650-651.
- 11.: Spring FA. Characterization of blood-group-active erythrocyte membrane glycoproteins with human antisera. Transfus Med. 1993;3:167-178.
- 12.: DeMarco M, Uhl L, Fields L, Pacini D, Gorlin JB, Kruskall MS. Hemolytic disease of the newbom due to the Scianna antibody, anti-Sc2. Transfusion. 1995;35:58-60.
- 13.: Rausen AR, Rosenfield RE, Alter AA, et al. A "new" infrequent red cell antigen, Rd (radin). Transfusion. 1967;7:336-342.
- 14.: Gassner C, Schmarda A, Kilga-Nogler S, et al. RhesusD/CE typing by polymerase chain reaction using sequence-specific primers. Transfusion. 1997;37:1020-1026.
- 15.: Wagner FF, Frohmajer A, Flegel WA. RHD positive haplotypes in D negative Europeans. BMC Genet. 2001;2:10.
- 16.: Ceppellini R, Siniscalco M, Smith CAB. The estimation of gene frequencies in a random-mating population. Ann Hum Genetics. 1955;20:97-114.
- 17.: Nielsen H, Engelbrecht J, Brunak S, von Heijne G. Identification of prokaryotic and eukaryotic signal peptides and prediction of their cleavage sites. Protein Engineering. 1997;10:1-6.
- 18.: Nielsen H, Krogh A. Prediction of signal peptides and signal anchors by a hidden Markov model. In: Anonymous. Proceedings of the Sixth International Conference on Intelligent Systems for Molecular Biology. AAAI Press, Menio Park, California; 1998:122-130.
- 19.: Bates PA, Kelley LA, MacCallum RM, Sternberg MJE. Enhancement of Protein Modelling by Human Intervention in Applying the Automatic Programs 3D-JIGSAW and 3D-PSSM. Proteins: Structure, Function and Genetics, Suppl. 2001;5:39-46.
- 20.: Bates PA, Stemberg MJE. Model Building by Comparison at CASP3: Using Expert Knowledge and Computer Automation. Proteins: Structure, Function and Genetics, Suppl. 1999;3:47-54.
- 21.: Fotinou C, Beauchamp J, Emsley P, et al. Structure of an Fab fragment against a C-terminal peptide of hCG at 2.0 A resolution. J Biol Chem. 1998;273:22515-22518.
- 22.: Xu H, Foltz L, Sha Y, et al. Cloning and characterization of human erythroid membrane-associated protein, human ERMAP. Blood Cells Mol Dis. 2001;76:2-4.
- 23.: Su YY, Gordon CT, Ye TZ, et al. Human ERMAP: An Erythroid Adhesion/Receptor Transmembrane Protein
Cloning and characterization of human erythroid membrane-associated protein, human ERMAP
Ermap, a gene coding for a novel erythroid specific adhesion/receptor membrane protein. Blood Cells Mol Dis. 2001;27:938-949.
- 24.: Devine P, Dawson FE, Motschman TL, et al. Serologic evidence that Scianna null (Sc:-1,-2) red cells lack multiple high-frequency antigens. Transfusion. 1988:28:346-349.
- 25.: Parsons SF, Mallinson G, Holmes CH, et al. The Lutheran blood group glycoprotein, another member of the immunoglobulin superfamily, is widely expressed in human tissues and is developmentally regulated in human liver. Proc Natl Acad Sci U S A. 1995;92:5496-5500.
- 26.: Bailly P, Hermand P, Callebaut I, et al. The LW blood group glycoprotein is homologous to intercellular adhesion molecules. Proc Natl Acad Sci USA. 199491:5306-5310.
- 27.: Spring FA, Holmes CH, Simpson KL, et al. The Oka blood group antigen is a marker for the M6 leukocyte activation antigen, the human homolog of OX-47 antigen, basigin and neurothelin, an immunoglobulin superfamily molecule that is widely expressed in human cells and tissues. Eur J Immunol. 1997;27:891-897.
- 28.: Joshi SR, Wagner FF, Vasantha K, Panjwani SR, Flegel WA. An *AQP1* null allele in an Indian woman with Co(a-b-) phenotype and high-titer anti-Co3 associated with mild HDN. Transfusion. 2001;41:1273-1278.
- 29.: King LS, Choi M, Fernandez PC, Cartron JP, Agre P. Defective urinary-concentrating ability due to a complete deficiency of aquaporin-1. N Engl J Med. 2001:345:175-179.

## Claims

1. A polynucleotide encoding
human ERMAP,
a variant of human ERMAP,
a fragment of human ERMAP, or
a fragment of said variant,
said polynucleotide carrying at least one mutation as compared to the nucleotide sequence as shown in SEQ ID NO:1.

2. A polynucleotide according to claim 1 wherein said mutation is a missense mutation causing an amino acid substitution in the extracellular portion of the ERMAP protein.

3. A polynucleotide according to claim 1 or 2 wherein said mutation is a missense mutation causing an amino acid substitution in position 26, 57 and/or 60 of the amino acid sequence of ERMAP.

4. A polynucleotide according to claim 3 wherein said amino acid substitution in position 26 is from His to Tyr, in position 57 from Gly to Arg, and/or in position 60 from Pro to Ala.

5. A polynucleotide according to claim 1 wherein said mutation is a deletion causing a shift in the reading frame of the ERMAP gene.

6. A polynucleotide according to anyone of claims 1 to 5 wherein said mutation occurs in nucleotide position 54, 76, 169, 178, 307 and/or 308.

7. A polynucleotide according to claim 6 wherein said mutation is a silent mutation in nucleotide position 54 from C to T, or a missense mutation in position 76 from C to T, in position 169 from G to A, and/or in position 178 from C to G.

8. A polynucleotide according to claim 6 wherein said mutation is a deletion of nucleotide position 307 and 308 of the ERMAP gene as shown in SEQ ID NO:1.

9. A polynucleotide according to anyone of claims 1 to 8 having a nucleotide sequence which is selected from the group consisting of SEQ ID NO:3, SEQ ID NO:5, SEQ ID NO:6, SEQ ID NO:8 and SEQ ID NO:10.

10. A vector comprising a polynucleotide according to anyone of claims 1 to 9.

11. A non-human host transformed or transfected with a vector according to claim 10.

12. A method of producing a polypeptide which comprises a variant of human ERMAP or a fragment thereof said method comprising culturing a host according to claim 11 under suitable conditions and isolating the polypeptide produced.

13. A polypeptide encoded by a polynucleotide according to anyone of claims 2 to 9 and/or obtainable by the method of claim 12.

14. An oligonucleotide capable of hybridizing under stringent conditions to a portion of a polynucleotide according to anyone of claims 1 to 9 comprising said at least one mutation or to the complementary portion thereof.

15. An oligonucleotide according to claim 14 wherein the oligonucleotide does not hybridize under stringent conditions to a polynucleotide having a sequence as shown in SEQ ID NO:1.

16. An antibody or aptamer or phage specifically binding to a polypeptide according to claim 13.

17. An antibody or aptamer or phage according to claim 16 which does not bind to wild type ERMAP.

18. An antibody or aptamer or phage specifically binding to wild type ERMAP but not to a polypeptide according to claim 13.

19. A method for testing for the presence of a polynucleotide encoding a Scianna antigen in a sample comprising detecting the presence or absence of at least one mutation in a polynucleotide representing the human ERMAP gene.

20. A method according to claim 19 comprising hybridizing an oligonucleotide according to claim 14 or 15 under stringent conditions to polynucleotides contained in the sample obtained from a human and detecting said hybridization.

21. A method according to claim 20 further comprising digesting the product of said hybridisation with a restriction enzyme and analysing the product(s) of said digestion.

22. A method according to claim 19 comprising determining the nucleic acid sequence of at least a portion of the polynucleotide representing the ERMAP gene.

23. A method according to claim 22 further comprising prior to determining said nucleic acid sequence, amplification of at least said portion of said polynucleotide.

24. A method according to claim 19 comprising carrying out an amplification reaction wherein at least one of the primers employed in said amplification reaction is an oligonucleotide according to claim 14 or 15.

25. A method for testing for the presence of a Scianna antigen in a sample comprising assaying a sample obtained from a human for specific binding to an antibody or aptamer or phage according to claim 16 or 17, or to an antibody or aptamer or phage according to claim 18.

26. A method according to anyone of claims 19 to 25 wherein said polynucleotide or proteinaceous material from said sample is fixed to a solid support, preferably to a chip.

27. The use of a polynucleotide according to anyone of claims 1 to 9, or of an oligonucleotide according to claim 14 or 15 or of an antibody or an aptamer or a phage according to anyone of claims 16 to 18 for the detection of a Scianna antigen and/or for the determination of the Scianna antigen type.

28. The use of cells from a proband, preferably red blood cells, which have been **characterized by** a method according to anyone of claims 19 to 26 for a serologic test.

29. Kit useful for determining Scianna antigen comprising
a) at least one oligonucleotide according to claim 14 or 15; and/or
b) at least one antibody or aptamer or phage according to anyone of claims 16 to 18.


SEQUENCE LISTING

<110> Biotest AG

<120> Scianna antigens

<130> Scianna

<140>
<141>

<160> 48

<170> PatentIn Ver. 2.1

<210> 1
<211> 1428
<212> DNA
<213> Homo sapiens

<400> 1
atggagatgg cgagttctgc tggctcctgg ctctctggct gcctcatccc tctcgtcttc 60
ctccggctgt ctgtgcatgt gtcaggccac gcaggggatg ccggcaagtt ccacgtggcc 120
ctactagggg gcacagccga gctgctctgc cctctctccc tctggcccgg gacggtaccc 180
aaggaggtga ggtggctgcg gtccccattc ccgcagcgtt cccaggctgt tcacatattc 240
cgggatggga aggaccagga tgaagatctg atgccggaat ataaggggag gacggtgcta 300
gtgagagatg cccaagaggg aagtgtcact ctgcagatcc ttgacgtgcg ccttgaggac 360
caagggtctt accgatgtct gatccaagtt ggaaatctga gtaaagagga caccgtgatc 420
ctgcaggttg cagccccatc tgtggggagt ctctccccct cagcagtggc tctggctgtg 480
atcctgcctg tcctggtact tctcatcatg gtgtgccttt gccttatctg gaagcaaaga 540
agagcaaaag aaaagcttct ctatgaacat gtgacggagg tggacaatct tctttcagac 600
catgctaaag aaaaaggaaa actccataaa gctgtcaaga aactccggag tgaactgaag 660
ttgaaaagag ctgcagcaaa ctcaggctgg agaagagccc ggttgcattt tgtggcagtg 720
accctggacc cagacacagc acatcccaaa ctcatccttt ctgaggacca aagatgtgta 780
aggcttggag acagacggca gcctgtacct gacaaccccc agagatttga tttcgttgtc 840
agcatcctag gctctgagta cttcacgact ggctgccact actgggaggt gtatgtggga 900
gacaagacca aatggattct tggagtatgt agtgagtcag tgagcaggaa ggggaaggtt 960
actgcctcac ctgccaatgg acactggctt ctgcgacaga gtcgtgggaa tgagtatgaa 1020
gctctcacat ccccgcagac ctccttccgc cttaaagagc ctccacggtg tgtggggatt 1080
ttcctggact atgaagcagg agtcatctct ttctacaatg tgaccaacaa gtcccacatc 1140
tttactttca cccacaattt ctctggcccc cttcgccctt tctttgaacc ttgccttcat 1200
gatggaggaa aaaacacagc acctctagtc atttgttcag aactacacaa atcagaggaa 1260
tcaattgtcc ccaggccaga agggaaaggc catgctaatg gagatgtgtc cctcaaggtg 1320
aactcttctt tactaccccc gaaggcccca gagctgaagg atataatcct gtccttgccc 1380
cctgaccttg gcccagccct tcaggagctc aaggctcctt ctttttag 1428

<210> 2

<211> 475
<212> PRT
<213> Homo sapiens

<400> 2
Met Glu Met Ala Ser Ser Ala Gly Ser Trp Leu Ser Gly Cys Leu Ile
1               5                   10                  15

Pro Leu Val Phe Leu Arg Leu Ser Val His Val Ser Gly His Ala Gly
            20                  25                  30

Asp Ala Gly Lys Phe His Val Ala Leu Leu Gly Gly Thr Ala Glu Leu
        35                  40                  45

Leu Cys Pro Leu Ser Leu Trp Pro Gly Thr Val Pro Lys Glu Val Arg
    50                  55                  60

Trp Leu Arg Ser Pro Phe Pro Gln Arg Ser Gln Ala Val His Ile Phe
65                  70                  75                  80

Arg Asp Gly Lys Asp Gln Asp Glu Asp Leu Met Pro Glu Tyr Lys Gly
                85                  90                  95

Arg Thr Val Leu Val Arg Asp Ala Gln Glu Gly Ser Val Thr Leu Gln
            100                 105                 110

Ile Leu Asp Val Arg Leu Glu Asp Gln Gly Ser Tyr Arg Cys Leu Ile
        115                 120                 125

Gln Val Gly Asn Leu Ser Lys Glu Asp Thr Val Ile Leu Gln Val Ala
    130                 135                 140

Ala Pro Ser Val Gly Ser Leu Ser Pro Ser Ala Val Ala Leu Ala Val
145                 150                 155                 160

Ile Leu Pro Val Leu Val Leu Leu Ile Met Val Cys Leu Cys Leu Ile
                165                 170                 175

Trp Lys Gln Arg Arg Ala Lys Glu Lys Leu Leu Tyr Glu His Val Thr
            180                 185                 190

Glu Val Asp Asn Leu Leu Ser Asp His Ala Lys Glu Lys Gly Lys Leu
        195                 200                 205

His Lys Ala Val Lys Lys Leu Arg Ser Glu Leu Lys Leu Lys Arg Ala
    210                 215                 220

Ala Ala Asn Ser Gly Trp Arg Arg Ala Arg Leu His Phe Val Ala Val

225                 230                 235                 240

Thr Leu Asp Pro Asp Thr Ala His Pro Lys Leu Ile Leu Ser Glu Asp
                245                 250                 255

Gln Arg Cys Val Arg Leu Gly Asp Arg Arg Gln Pro Val Pro Asp Asn
            260                 265                 270

Pro Gln Arg Phe Asp Phe Val Val Ser Ile Leu Gly Ser Glu Tyr Phe
        275                 280                 285

Thr Thr Gly Cys His Tyr Trp Glu Val Tyr Val Gly Asp Lys Thr Lys
    290                 295                 300

Trp Ile Leu Gly Val Cys Ser Glu Ser Val Ser Arg Lys Gly Lys Val
305                 310                 315                 320

Thr Ala Ser Pro Ala Asn Gly His Trp Leu Leu Arg Gln Ser Arg Gly
                325                 330                 335

Asn Glu Tyr Glu Ala Leu Thr Ser Pro Gln Thr Ser Phe Arg Leu Lys
            340                 345                 350

Glu Pro Pro Arg Cys Val Gly Ile Phe Leu Asp Tyr Glu Ala Gly Val
        355                 360                 365

Ile Ser Phe Tyr Asn Val Thr Asn Lys Ser His Ile Phe Thr Phe Thr
    370                 375                 380

His Asn Phe Ser Gly Pro Leu Arg Pro Phe Phe Glu Pro Cys Leu His
385                 390                 395                 400

Asp Gly Gly Lys Asn Thr Ala Pro Leu Val Ile Cys Ser Glu Leu His
                405                 410                 415

Lys Ser Glu Glu Ser Ile Val Pro Arg Pro Glu Gly Lys Gly His Ala
            420                 425                 430

Asn Gly Asp Val Ser Leu Lys Val Asn Ser Ser Leu Leu Pro Pro Lys
        435                 440                 445

Ala Pro Glu Leu Lys Asp Ile Ile Leu Ser Leu Pro Pro Asp Leu Gly
    450                 455                 460

Pro Ala Leu Gln Glu Leu Lys Ala Pro Ser Phe
465                 470                 475

<210> 3
<211> 1428
<212> DNA
<213> Homo sapiens

<400> 3
atggagatgg cgagttctgc tggctcctgg ctctctggct gcctcatccc tctcgtcttc 60
ctccggctgt ctgtgtatgt gtcaggccac gcaggggatg ccggcaagtt ccacgtggcc 120
ctactagggg gcacagccga gctgctctgc cctctctccc tctggcccgg gacggtaccc 180
aaggaggtga ggtggctgcg gtccccattc ccgcagcgtt cccaggctgt tcacatattc 240
cgggatggga aggaccagga tgaagatctg atgccggaat ataaggggag gacggtgcta 300
gtgagagatg cccaagaggg aagtgtcact ctgcagatcc ttgacgtgcg ccttgaggac 360
caagggtctt accgatgtct gatccaagtt ggaaatctga gtaaagagga caccgtgatc 420
ctgcaggttg cagccccatc tgtggggagt ctctccccct cagcagtggc tctggctgtg 480
atcctgcctg tcctggtact tctcatcatg gtgtgccttt gccttatctg gaagcaaaga 540
agagcaaaag aaaagcttct ctatgaacat gtgacggagg tggacaatct tctttcagac 600
catgctaaag aaaaaggaaa actccataaa gctgtcaaga aactccggag tgaactgaag 660
ttgaaaagag ctgcagcaaa ctcaggctgg agaagagccc ggttgcattt tgtggcagtg 720
accctggacc cagacacagc acatcccaaa ctcatccttt ctgaggacca aagatgtgta 780
aggcttggag acagacggca gcctgtacct gacaaccccc agagatttga tttcgttgtc 840
agcatcctag gctctgagta cttcacgact ggctgccact actgggaggt gtatgtggga 900
gacaagacca aatggattct tggagtatgt agtgagtcag tgagcaggaa ggggaaggtt 960
actgcctcac ctgccaatgg acactggctt ctgcgacaga gtcgtgggaa tgagtatgaa 1020
gctctcacat ccccgcagac ctccttccgc cttaaagagc ctccacggtg tgtggggatt 1080
ttcctggact atgaagcagg agtcatctct ttctacaatg tgaccaacaa gtcccacatc 1140
tttactttca cccacaattt ctctggcccc cttcgccctt tctttgaacc ttgccttcat 1200
gatggaggaa aaaacacagc acctctagtc atttgttcag aactacacaa atcagaggaa 1260
tcaattgtcc ccaggccaga agggaaaggc catgctaatg gagatgtgtc cctcaaggtg 1320
aactcttctt tactaccccc gaaggcccca gagctgaagg atataatcct gtccttgccc 1380
cctgaccttg gcccagccct tcaggagctc aaggctcctt ctttttag 1428

<210> 4
<211> 475
<212> PRT
<213> Homo sapiens

<400> 4


<210> 5
<211> 1428
<212> DNA
<213> Homo sapiens

<400> 5
atggagatgg cgagttctgc tggctcctgg ctctctggct gcctcatccc tcttgtcttc 60
ctccggctgt ctgtgtatgt gtcaggccac gcaggggatg ccggcaagtt ccacgtggcc 120
ctactagggg gcacagccga gctgctctgc cctctctccc tctggcccgg gacggtaccc 180
aaggaggtga ggtggctgcg gtccccattc ccgcagcgtt cccaggctgt tcacatattc 240
cgggatggga aggaccagga tgaagatctg atgccggaat ataaggggag gacggtgcta 300
gtgagagatg cccaagaggg aagtgtcact ctgcagatcc ttgacgtgcg ccttgaggac 360
caagggtctt accgatgtct gatccaagtt ggaaatctga gtaaagagga caccgtgatc 420
ctgcaggttg cagccccatc tgtggggagt ctctccccct cagcagtggc tctggctgtg 480
atcctgcctg tcctggtact tctcatcatg gtgtgccttt gccttatctg gaagcaaaga 540

agagcaaaag aaaagcttct ctatgaacat gtgacggagg tggacaatct tctttcagac 600
catgctaaag aaaaaggaaa actccataaa gctgtcaaga aactccggag tgaactgaag 660
ttgaaaagag ctgcagcaaa ctcaggctgg agaagagccc ggttgcattt tgtggcagtg 720
accctggacc cagacacagc acatcccaaa ctcatccttt ctgaggacca aagatgtgta 780
aggcttggag acagacggca gcctgtacct gacaaccccc agagatttga tttcgttgtc 840
agcatcctag gctctgagta cttcacgact ggctgccact actgggaggt gtatgtggga 900
gacaagacca aatggattct tggagtatgt agtgagtcag tgagcaggaa ggggaaggtt 960
actgcctcac ctgccaatgg acactggctt ctgcgacaga gtcgtgggaa tgagtatgaa 1020
gctctcacat ccccgcagac ctccttccgc cttaaagagc ctccacggtg tgtggggatt 1080
ttcctggact atgaagcagg agtcatctct ttctacaatg tgaccaacaa gtcccacatc 1140
tttactttca cccacaattt ctctggcccc cttcgccctt tctttgaacc ttgccttcat 1200
gatggaggaa aaaacacagc acctctagtc atttgttcag aactacacaa atcagaggaa 1260
tcaattgtcc ccaggccaga agggaaaggc catgctaatg gagatgtgtc cctcaaggtg 1320
aactcttctt tactaccccc gaaggcccca gagctgaagg atataatcct gtccttgccc 1380
cctgaccttg gcccagccct tcaggagctc aaggctcctt ctttttag 1428

<210> 6
<211> 1428
<212> DNA
<213> Homo sapiens

<400> 6
atggagatgg cgagttctgc tggctcctgg ctctctggct gcctcatccc tctcgtcttc 60
ctccggctgt ctgtgcatgt gtcaggccac gcaggggatg ccggcaagtt ccacgtggcc 120
ctactagggg gcacagccga gctgctctgc cctctctccc tctggcccag gacggtaccc 180
aaggaggtga ggtggctgcg gtccccattc ccgcagcgtt cccaggctgt tcacatattc 240
cgggatggga aggaccagga tgaagatctg atgccggaat ataaggggag gacggtgcta 300
gtgagagatg cccaagaggg aagtgtcact ctgcagatcc ttgacgtgcg ccttgaggac 360
caagggtctt accgatgtct gatccaagtt ggaaatctga gtaaagagga caccgtgatc 420
ctgcaggttg cagccccatc tgtggggagt ctctccccct cagcagtggc tctggctgtg 480
atcctgcctg tcctggtact tctcatcatg gtgtgccttt gccttatctg gaagcaaaga 540
agagcaaaag aaaagcttct ctatgaacat gtgacggagg tggacaatct tctttcagac 600
catgctaaag aaaaaggaaa actccataaa gctgtcaaga aactccggag tgaactgaag 660
ttgaaaagag ctgcagcaaa ctcaggctgg agaagagccc ggttgcattt tgtggcagtg 720
accctggacc cagacacagc acatcccaaa ctcatccttt ctgaggacca aagatgtgta 780
aggcttggag acagacggca gcctgtacct gacaaccccc agagatttga tttcgttgtc 840
agcatcctag gctctgagta cttcacgact ggctgccact actgggaggt gtatgtggga 900
gacaagacca aatggattct tggagtatgt agtgagtcag tgagcaggaa ggggaaggtt 960
actgcctcac ctgccaatgg acactggctt ctgcgacaga gtcgtgggaa tgagtatgaa 1020
gctctcacat ccccgcagac ctccttccgc cttaaagagc ctccacggtg tgtggggatt 1080
ttcctggact atgaagcagg agtcatctct ttctacaatg tgaccaacaa gtcccacatc 1140
tttactttca cccacaattt ctctggcccc cttcgccctt tctttgaacc ttgccttcat 1200
gatggaggaa aaaacacagc acctctagtc atttgttcag aactacacaa atcagaggaa 1260
tcaattgtcc ccaggccaga agggaaaggc catgctaatg gagatgtgtc cctcaaggtg 1320
aactcttctt tactaccccc gaaggcccca gagctgaagg atataatcct gtccttgccc 1380
cctgaccttg gcccagccct tcaggagctc aaggctcctt ctttttag 1428

<210> 7
<211> 475
<212> PRT
<213> Homo sapiens

<400> 7

<210> 8
<211> 1426
<212> DNA
<213> Homo sapiens

<400> 8
   atggagatgg cgagttctgc tggctcctgg ctctctggct gcctcatccc tcttgtcttc 60
   ctccggctgt ctgtgtatgt gtcaggccac gcaggggatg ccggcaagtt ccacgtggcc 120
   ctactagggg gcacagccga gctgctctgc cctctctccc tctggcccgg gacggtaccc 180
   aaggaggtga ggtggctgcg gtccccattc ccgcagcgtt cccaggctgt tcacatattc 240
   cgggatggga aggaccagga tgaagatctg atgccggaat ataaggggag gacggtgcta 300
   gtgagatgcc caagagggaa gtgtcactct gcagatcctt gacgtgcgcc ttgaggacca 360
   agggtcttac cgatgtctga tccaagttgg aaatctgagt aaagaggaca ccgtgatcct 420
   gcaggttgca gccccatctg tggggagtct ctccccctca gcagtggctc tggctgtgat 480
   cctgcctgtc ctggtacttc tcatcatggt gtgcctttgc cttatctgga agcaaagaag 540
   agcaaaagaa aagcttctct atgaacatgt gacggaggtg gacaatcttc tttcagacca 600
   tgctaaagaa aaaggaaaac tccataaagc tgtcaagaaa ctccggagtg aactgaagtt 660
   gaaaagagct gcagcaaact caggctggag aagagcccgg ttgcattttg tggcagtgac 720
   cctggaccca gacacagcac atcccaaact catcctttct gaggaccaaa gatgtgtaag 780
   gcttggagac agacggcagc ctgtacctga caacccccag agatttgatt tcgttgtcag 840
   catcctaggc tctgagtact tcacgactgg ctgccactac tgggaggtgt atgtgggaga 900
   caagaccaaa tggattcttg gagtatgtag tgagtcagtg agcaggaagg ggaaggttac 960
   tgcctcacct gccaatggac actggcttct gcgacagagt cgtgggaatg agtatgaagc 1020
   tctcacatcc ccgcagacct ccttccgcct taaagagcct ccacggtgtg tggggatttt 1080
   cctggactat gaagcaggag tcatctcttt ctacaatgtg accaacaagt cccacatctt 1140
   tactttcacc cacaatttct ctggccccct tcgccctttc tttgaacctt gccttcatga 1200
   tggaggaaaa aacacagcac ctctagtcat ttgttcagaa ctacacaaat cagaggaatc 1260
   aattgtcccc aggccagaag ggaaaggcca tgctaatgga gatgtgtccc tcaaggtgaa 1320
   ctcttcttta ctacccccga aggccccaga gctgaaggat ataatcctgt ccttgccccc 1380
   tgaccttggc ccagcccttc aggagctcaa ggctccttct ttttag 1426

<210> 9
<211> 113
<212> PRT
<213> Homo sapiens

<400> 9

<210> 10
<211> 1428
<212> DNA
<213> Homo sapiens

<400> 10
atggagatgg cgagttctgc tggctcctgg ctctctggct gcctcatccc tctcgtcttc 60
ctccggctgt ctgtgcatgt gtcaggccac gcaggggatg ccggcaagtt ccacgtggcc 120
ctactagggg gcacagccga gctgctctgc cctctctccc tctggcccgg gacggtagcc 180
aaggaggtga ggtggctgcg gtccccattc ccgcagcgtt cccaggctgt tcacatattc 240
cgggatggga aggaccagga tgaagatctg atgccggaat ataaggggag gacggtgcta 300
gtgagagatg cccaagaggg aagtgtcact ctgcagatcc ttgacgtgcg ccttgaggac 360
caagggtctt accgatgtct gatccaagtt ggaaatctga gtaaagagga caccgtgatc 420
ctgcaggttg cagccccatc tgtggggagt ctctccccct cagcagtggc tctggctgtg 480
atcctgcctg tcctggtact tctcatcatg gtgtgccttt gccttatctg gaagcaaaga 540
agagcaaaag aaaagcttct ctatgaacat gtgacggagg tggacaatct tctttcagac 600
catgctaaag aaaaaggaaa actccataaa gctgtcaaga aactccggag tgaactgaag 660
ttgaaaagag ctgcagcaaa ctcaggctgg agaagagccc ggttgcattt tgtggcagtg 720
accctggacc cagacacagc acatcccaaa ctcatccttt ctgaggacca aagatgtgta 780
aggcttggag acagacggca gcctgtacct gacaaccccc agagatttga tttcgttgtc 840
agcatcctag gctctgagta cttcacgact ggctgccact actgggaggt gtatgtggga 900
gacaagacca aatggattct tggagtatgt agtgagtcag tgagcaggaa ggggaaggtt 960
actgcctcac ctgccaatgg acactggctt ctgcgacaga gtcgtgggaa tgagtatgaa 1020
gctctcacat ccccgcagac ctccttccgc cttaaagagc ctccacggtg tgtggggatt 1080
ttcctggact atgaagcagg agtcatctct ttctacaatg tgaccaacaa gtcccacatc 1140
tttactttca cccacaattt ctctggcccc cttcgccctt tctttgaacc ttgccttcat 1200
gatggaggaa aaaacacagc acctctagtc atttgttcag aactacacaa atcagaggaa 1260
tcaattgtcc ccaggccaga agggaaaggc catgctaatg gagatgtgtc cctcaaggtg 1320
aactcttctt tactaccccc gaaggcccca gagctgaagg atataatcct gtccttgccc 1380
cctgaccttg gcccagccct tcaggagctc aaggctcctt ctttttag 1428

<210> 11
<211> 475
<212> PRT
<213> Homo sapiens

<400> 11
   Met Glu Met Ala Ser Ser Ala Gly Ser Trp Leu Ser Gly Cys Leu Ile
   1               5                   10                  15
   Pro Leu Val Phe Leu Arg Leu Ser Val His Val Ser Gly His Ala Gly
               20                  25                  30
   Asp Ala Gly Lys Phe His Val Ala Leu Leu Gly Gly Thr Ala Glu Leu
           35                  40                  45
   Leu Cys Pro Leu Ser Leu Trp Pro Gly Thr Val Ala Lys Glu Val Arg
       50                  55                  60
   Trp Leu Arg Ser Pro Phe Pro Gln Arg Ser Gln Ala Val His Ile Phe
   65                  70                  75                  80
   Arg Asp Gly Lys Asp Gln Asp Glu Asp Leu Met Pro Glu Tyr Lys Gly
                   85                  90                  95
   Arg Thr Val Leu Val Arg Asp Ala Gln Glu Gly Ser Val Thr Leu Gln
               100                 105                 110
   Ile Leu Asp Val Arg Leu Glu Asp Gln Gly Ser Tyr Arg Cys Leu Ile
           115                 120                 125
   Gln Val Gly Asn Leu Ser Lys Glu Asp Thr Val Ile Leu Gln Val Ala
       130                 135                 140
   Ala Pro Ser Val Gly Ser Leu Ser Pro Ser Ala Val Ala Leu Ala Val
   145                 150                 155                 160
   Ile Leu Pro Val Leu Val Leu Leu Ile Met Val Cys Leu Cys Leu Ile
                   165                 170                 175
   Trp Lys Gln Arg Arg Ala Lys Glu Lys Leu Leu Tyr Glu His Val Thr
               180                 185                 190
   Glu Val Asp Asn Leu Leu Ser Asp His Ala Lys Glu Lys Gly Lys Leu
           195                 200                 205
   His Lys Ala Val Lys Lys Leu Arg Ser Glu Leu Lys Leu Lys Arg Ala
       210                 215                 220
   Ala Ala Asn Ser Gly Trp Arg Arg Ala Arg Leu His Phe Val Ala Val
   225                 230                 235                 240
   Thr Leu Asp Pro Asp Thr Ala His Pro Lys Leu Ile Leu Ser Glu Asp
                   245                 250                 255
   Gln Arg Cys Val Arg Leu Gly Asp Arg Arg Gln Pro Val Pro Asp Asn
               260                 265                 270
   Pro Gln Arg Phe Asp Phe Val Val Ser Ile Leu Gly Ser Glu Tyr Phe
           275                 280                 285
   Thr Thr Gly Cys His Tyr Trp Glu Val Tyr Val Gly Asp Lys Thr Lys
       290                 295                 300
   Trp Ile Leu Gly Val Cys Ser Glu Ser Val Ser Arg Lys Gly Lys Val
   305                 310                 315                 320
   Thr Ala Ser Pro Ala Asn Gly His Trp Leu Leu Arg Gln Ser Arg Gly
                   325                 330                 335
   Asn Glu Tyr Glu Ala Leu Thr Ser Pro Gln Thr Ser Phe Arg Leu Lys
               340                 345                 350
   Glu Pro Pro Arg Cys Val Gly Ile Phe Leu Asp Tyr Glu Ala Gly Val
           355                 360                 365
   Ile Ser Phe Tyr Asn Val Thr Asn Lys Ser His Ile Phe Thr Phe Thr
       370                 375                 380
   His Asn Phe Ser Gly Pro Leu Arg Pro Phe Phe Glu Pro Cys Leu His
   385                 390                 395                 400
   Asp Gly Gly Lys Asn Thr Ala Pro Leu Val Ile Cys Ser Glu Leu His
                   405                 410                 415
   Lys Ser Glu Glu Ser Ile Val Pro Arg Pro Glu Gly Lys Gly His Ala
               420                 425                 430
   Asn Gly Asp Val Ser Leu Lys Val Asn Ser Ser Leu Leu Pro Pro Lys
           435                 440                 445
   Ala Pro Glu Leu Lys Asp Ile Ile Leu Ser Leu Pro Pro Asp Leu Gly
       450                 455                 460
   Pro Ala Leu Gln Glu Leu Lys Ala Pro Ser Phe
   465                 470                 475

<210> 12
<211> 20
<212> DNA
<213> Artificial Sequence

<220>
<223> Description of Artificial Sequence: primer

<400> 12
catacagccg gaggaagacg                                                    20

<210> 13
<211> 20
<212> DNA
<213> Artificial Sequence

<220>
<223> Description of Artificial Sequence: primer

<400> 13
catacagccg gaggaagaca                                                    20

<210> 14
<211> 26
<212> DNA
<213> Artificial Sequence

<220>
<223> Description of Artificial Sequence: primer

<400> 14
gatcagaaac tcctacctga cacttg                                             26

<210> 15
<211> 26
<212> DNA
<213> Artificial Sequence

<220>
<223> Description of Artificial Sequence: primer

<400> 15
gatcagaaac tcctacctga cactta                                             26

<210> 16
<211> 22
<212> DNA
<213> Artificial Sequence

<220>
<223> Description of Artificial Sequence: primer

<400> 16
gcagccacct cacctccttt gc                                                22

<210> 17
<211> 22
<212> DNA
<213> Artificial Sequence

<220>
<223> Description of Artificial Sequence: primer

<400> 17
tcacctcctt gggtaccgta ct                                                22

<210> 18
<211> 22
<212> DNA
<213> Artificial Sequence

<220>
<223> Description of Artificial Sequence: primer

<400> 18
tcacctcctt gggtaccgta cc                                                22

<210> 19
<211> 25
<212> DNA
<213> Artificial Sequence

<220>
<223> Description of Artificial Sequence: primer

<400> 19
cctccgtcac atgttcatag agaag                                             25

<210> 20
<211> 22
<212> DNA
<213> Artificial Sequence

<220>
<223> Description of Artificial Sequence: primer

<400> 20
gtgtgtgggg attttcctgg ac                                                22

<210> 21
<211> 20
<212> DNA
<213> Artificial Sequence

<220>
<223> Description of Artificial Sequence: primer

<400> 21
gccaggcgac tgaagtccag                                                   20

<210> 22
<211> 23
<212> DNA
<213> Artificial Sequence

<220>
<223> Description of Artificial Sequence: primer

<400> 22
gggacttgtt ggtcacattg tag                                               23

<210> 23
<211> 28
<212> DNA
<213> Artificial Sequence

<220>
<223> Description of Artificial Sequence: primer

<400> 23
gtaaggagaa aggggattat ttctctac                                          28

<210> 24
<211> 28
<212> DNA
<213> Artificial Sequence

<220>
<223> Description of Artificial Sequence: primer

<400> 24
cacccttgaa aatgttcttg tgcgtaag                                    28

<210> 25
<211> 27
<212> DNA
<213> Artificial Sequence

<220>
<223> Description of Artificial Sequence: primer

<400> 25
cataaggagg aagtgctctg agatatg                                     27

<210> 26
<211> 26
<212> DNA
<213> Artificial Sequence

<220>
<223> Description of Artificial Sequence: primer

<400> 26
ggcaagtaaa aactacactg cagatg                                      26

<210> 27
<211> 21
<212> DNA
<213> Artificial Sequence

<220>
<223> Description of Artificial Sequence: primer

<400> 27
caccagcctg agctccctta c                                           21

<210> 28
<211> 28
<212> DNA
<213> Artificial Sequence

<220>
<223> Description of Artificial Sequence: primer

<400> 28
gtggattctt gaaattccca tatatccc                                    28


<210> 29
<211> 26
<212> DNA
<213> Artificial Sequence

<220>
<223> Description of Artificial Sequence: primer

<400> 29
ggatatatgt gtgcatgtgt gtacag                                      26


<210> 30
<211> 20
<212> DNA
<213> Artificial Sequence

<220>
<223> Description of Artificial Sequence: primer

<400> 30
gtccccatgg cttgaattac                                             20


<210> 31
<211> 25
<212> DNA
<213> Artificial Sequence

<220>
<223> Description of Artificial Sequence: primer

<400> 31
gagtcaagcg attacttcag ctcac                                       25

<210> 32
<211> 20
<212> DNA
<213> Artificial Sequence

<220>
<223> Description of Artificial Sequence: primer

<400> 32
gcctcctctt tgctctgttc                                                    20


<210> 33
<211> 21
<212> DNA
<213> Artificial Sequence

<220>
<223> Description of Artificial Sequence: primer

<400> 33
gcctcggctc ctgggttata c                                                  21


<210> 34
<211> 22
<212> DNA
<213> Artificial Sequence

<220>
<223> Description of Artificial Sequence: primer

<400> 34
tctgtcacgc caagcccatc ag                                                 22


<210> 35
<211> 20
<212> DNA
<213> Artificial Sequence

<220>
<223> Description of Artificial Sequence: primer

<400> 35
agcccgcttg tgctgtctcc                                                    20

<210> 36
<211> 21
<212> DNA
<213> Artificial Sequence

<220>
<223> Description of Artificial Sequence: primer

<400> 36
ctcatccctt cccaagcttt c                                                21

<210> 37
<211> 20
<212> DNA
<213> Artificial Sequence

<220>
<223> Description of Artificial Sequence: primer

<400> 37
ctcccagttg gccttgtctc                                                  20

<210> 38
<211> 25
<212> DNA
<213> Artificial Sequence

<220>
<223> Description of Artificial Sequence: primer

<400> 38
cattatgtct tttggggttc tgttc                                            25

<210> 39
<211> 21
<212> DNA
<213> Artificial Sequence

<220>
<223> Description of Artificial Sequence: primer

<400> 39
ccatctgagg catgccagaa g                                                21

<210> 40
<211> 19
<212> DNA
<213> Artificial Sequence

<220>
<223> Description of Artificial Sequence: primer

<400> 40
gacatctccc tgaggtcag                                                    19


<210> 41
<211> 22
<212> DNA
<213> Artificial Sequence

<220>
<223> Description of Artificial Sequence: primer

<400> 41
atgcccgctt acctgtagtg ac                                                22


<210> 42
<211> 21
<212> DNA
<213> Artificial Sequence

<220>
<223> Description of Artificial Sequence: primer

<400> 42
gggaggtgga ggtgaagata g                                                 21


<210> 43
<211> 20
<212> DNA
<213> Artificial Sequence

<220>
<223> Description of Artificial Sequence: primer

<400> 43
gagtgatgag gctgccacag                                                   20

<210> 44
<211> 24
<212> DNA
<213> Artificial Sequence

<220>
<223> Description of Artificial Sequence: primer

<400> 44
cattatggga gggcctctac atag                                          24


<210> 45
<211> 26
<212> DNA
<213> Artificial Sequence

<220>
<223> Description of Artificial Sequence: primer

<400> 45
gggtatagct attgagtatc tgtctg                                        26


<210> 46
<211> 26
<212> DNA
<213> Artificial Sequence

<220>
<223> Description of Artificial Sequence: primer

<400> 46
ctaaaatggg ctaatgacag tgatgg                                        26


<210> 47
<211> 24
<212> DNA
<213> Artificial Sequence

<220>
<223> Description of Artificial Sequence: primer

<400> 47
acagtgatgg cagacctaag attc                                          24

<210> 48
<211> 30
<212> DNA
<213> Artificial Sequence

<220>
<223> Description of Artificial Sequence: primer

<400> 48
tcactgtgat agttaatctt atgtatccac                                        30